# FASCICULE DE BREVET EUROPEEN

(11) **EP 0 850 909 B1**
(45) Date de publication et mention de la délivrance du brevet: **21.03.2001**
(21) Numéro de dépôt: 97403043.9
(22) Date de dépôt: 15.12.1997
(51) Int. Cl.: C07C 65/28, C07C 69/76, C07C 235/06, C07C 235/42, C07C 323/62, C07C 43/23, C07C 47/575, C07D 213/79, C07D 213/80, A61K 7/06, A61K 7/48, A61K 31/085, A61K 31/11, A61K 31/165, A61K 31/19, A61K 31/235, A61K 31/44

(54) **Composés stilbéniques à groupement adamantyl, compositions les contenant et utilisations**
Stilbenverbindungen mit Adamantylgruppe, diese enthaltende Zusammensetzungen und deren Verwendung
Stilbene compounds with adamantyl group, compositions containing the same and uses

(30) Priorité: 31.12.1996 FR 9616311
(43) Date de publication de la demande: 01.07.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: Bernardon, Jean-Michel, 06650 Le Rouret (FR)
(74) Mandataire: Tezier Herman, Béatrice

(56) Documents cités:
- EP-A- 0 776 881
- EP-A- 0 776 885
- B. CHARPENTIER, ET AL.: "Synthesis, structure-affinity relationships, and biological activities of ligands binding to retinoic acid receptor subtypes" JOURNAL OF MEDICINAL CHEMISTRY, vol. 38, no. 26, 22 décembre 1995, WASHINGTON, DC, US, pages 4993-5006, XP002026831
- SUN, SHI-YONG ET AL: "Differential effects of synthetic nuclear retinoid receptor-selective retinoids on the growth of human non-small cell lung carcinoma cells" CANCER RES. (1997), 57(21), 4931-4939 CODEN: CNREA8;ISSN: 0008-5472, 1997, XP002062312

## Description

L'invention concerne, à titre de produits industriels nouveaux et utiles, des composés stilbéniques à groupement adamantyl. Elle concerne également l'utilisation de ces nouveaux composés dans des compositions pharmaceutiques destinées à un usage en médecine humaine ou vétérinaire, ou bien encore dans des compositions cosmétiques.

La publication de B. Charpentier et al., J. Med. Chem., vol.38, n°26, 4993-5006 (1995) décrit de tels composés stilbéniques ayant des activités biologiques de type agoniste RAR. Il n'est toutefois pas suggéré que des composés proches, qui font l'objet de la présente invention, puissent avoir une activité antagoniste RAR.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Les composés selon l'invention peuvent être représentés par la formule générale (I) suivante : 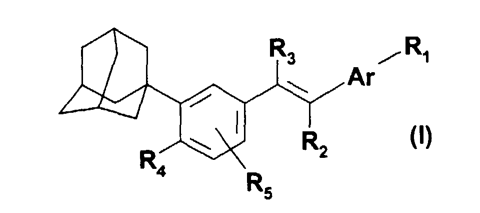 dans laquelle:
- R₁ représente
   (i) le radical -CH₃,
   (ii) le radical -CH₂-O-R₆,
   (iii) le radical -O-R₆,
   (iv) le radical -CO-R₇,
   les radicaux R₆ et R₇ ayant les significations données ci-après,
- Ar représente un radical choisi parmi les radicaux de formules (a) à (f) suivantes : 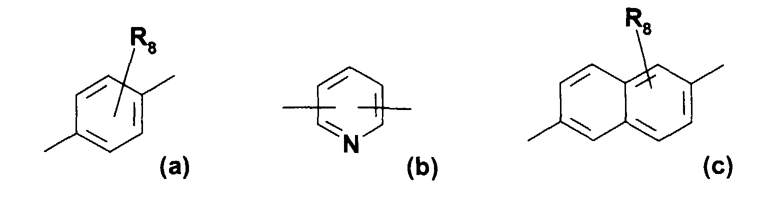 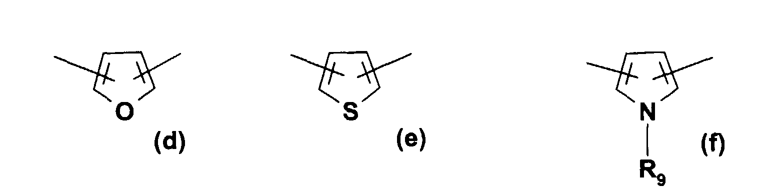 R₈ et R₉ ayant les significations données ci-après
- R₂ et R₃ , identiques ou différents, représentent un atome d'hydrogène, un radical alkyle inférieur,
- R₄ représente le radical -(X)m-(CH₂)n-Y-(CH₂)p-R₁₀
les valeurs m, n et p et les radicaux X, Y et R₁₀ ayant les significations données ci-après,
- R₅ représente un atome d'hydrogène, d'halogène, un radical alkyle inférieur, un radical -O-R₆,
- R₆ représente un atome d'hydrogène, un radical alkyle inférieur ou un radical -CO-R₁₁,
- R₇ représente un atome d'hydrogène, un radical alkyle inférieur, un radical -OR₁₂ ou un radical 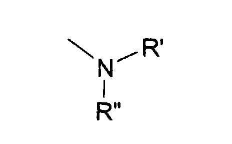 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore pris ensemble, forment un hétérocycle,
étant entendu que dans tout ce qui précède :
- m est un nombre entier égal à 0 ou 1,
- n est un nombre entier compris inclusivement entre 1 et 6,
- p est un nombre entier compris inclusivement entre 1 et 6,
- X représente O ou S(O)q,
- Y représente O, S(O)q ou N-R₉,
- q est un nombre entier compris inclusivement entre 0 et 2,
- R₈ représente un atome d'hydrogène, d'halogène, un radical alkyle inférieur ou un radical -O-R₆,
- R₉ représente un atome d'hydrogène, un radical alkyle inférieur ou un radical -CO-R₁₁,
- R₁₀ représente un radical mono ou polyhydroxyalkyle dont les hydroxy sont éventuellement protégés sous forme de méthoxy, éthoxy ou d'acétoxy ou d'acétonide, un radical -CO-R₇, un radical aryle ou aralkyle éventuellement substitué,
- R₁₁ représente un radical alkyle inférieur,
- R₁₂ représente un atome d'hydrogène, un radical alkyle, un radical alkényle, un radical mono ou polyhydroxyalkyle dont les hydroxy sont éventuellement protégés sous forme de méthoxy, éthoxy ou d'acétoxy ou d'acétonide, un radical aryle ou aralkyle éventuellement substitué ou un reste de sucre ou un reste d'aminoacide ou de peptide,
et les isomères optiques et géométriques desdits composés de formule (I) ainsi que leurs sels.

Lorsque les composés selon l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique. Lorsque les composés selon l'invention se présentent sous forme de sels par addition d'une base, il s'agit préférentiellment de sels d'un métal alcalin ou alcalino-terreux ou encore de zinc ou d'une amine organique.

Selon la présente invention, on entend par radical alkyle un radical linéaire ou ramifié éventuellement substitué par un ou plusieurs atomes d'halogène ayant de 1 à 20, de préférence de 1 à 12, atomes de carbone, avantageusement les radicaux méthyle, éthyle, isopropyle, butyle, tertiobutyle, hexyle, nonyle et dodécyle. Losqu'il est inférieur, le radical alkyl comprend généralement de 1 à 10 atomes de carbone, de préférence de 1 à 6 atomes de carbone. On peut citer, comme radical alkyle inférieur, les radicaux méthyle, éthyle, propyle, isopropyle, tertiobutyle, hexyle.

Parmi les radicaux alkyle linéaire ayant de 1 à 20 atomes de carbone, on peut citer notamment les radicaux méthyle, éthyle, propyle, 2-éthyl-hexyle, octyle, dodécyle, hexadécyle et octadécyl.

Parmi les radicaux alkyle ramifié ayant de 1 à 20 atomes de carbone, on peut citer notamment les radicaux 2-méthylbutyle, 2-méthylpentyle, 1-méthylhexyle, 3-méthylheptyle.

Par radical alkényle, on entend un radical ayant de 2 à 20 atomes de carbone linéaire ou ramifié comportant une ou plusieurs doubles liaisons.

Parmi les radicaux alkényle, on préfère un radical contenant de 2 à 5 atomes de carbone et présentant une ou plusieurs insaturations éthyléniques, tel que plus particulièrement le radical allyle.

Par radical monohydroxyalkyle ou polyhydroxyalkyle, on doit entendre un radical contenant de 1 à 6 atomes de carbone et de 1 à 5 groupes hydroxyles.

Parmi les radicaux monohydroxyalkyle, on préfère un radical contenant de préférence 1 ou 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on préfère un radical phényle éventuellement substitué par au moins un atome d'halogène, un radical hydroxyle, un radical alkyle, une fonction nitro, un groupe méthoxy ou une fonction amine éventuellement substituée.

Parmi les radicaux aralkyle, on préfère le radical benzyle ou phénéthyle éventuellement substitué par au moins un atome d'halogène, un radical hydroxyle, une fonction nitro ou un groupe méthoxy.

Par reste de sucre, on entend un reste dérivant notamment de glucose, de galactose ou de mannose, ou bien encore de l'acide glucuronique.

Par reste d'aminoacide, on entend notamment un reste dérivant de l'un des acides aminés tels que la lysine, la glycine ou l'acide aspartique, et par reste de peptide on entend plus particulièrement un reste de dipeptide ou de tripeptide résultant de la combinaison d'acides aminés.

Par hétérocycle, on entend de préférence un radical pipéridino, morpholino, pyrrolidino ou pipérazino, éventuellement substitué en position 4 par un radical alkyle en C₁-C₆ ou polyhydroxyalkyle tels que définis ci-dessus.

Lorsque les radicaux R₅ et R₈ représentent un atome d'halogène, celui-ci est de préférence un atome de fluor, de brome ou de chlore.

Parmi les composés de formule (I) ci-dessus rentrant dans le cadre de la présente invention, on peut notamment citer les suivants:
4-[(E)-2-(3-(1-adamantyl)-4-méthoxyéthoxyméthoxyphényl)éthényl]benzoate d'éthyle.
Acide 4-[(E)-2-(3-(1-adamantyl)-4-methoxyethoxymethoxyphényl) éthényl]benzoïque.
4-[(E)-2-(3-(1-adamantyl)-4-méthoxyéthoxyméthoxyphényl)-1-propényl] benzoate d'éthyle.
Acide 4-[(E)-2-(3-(1-adamantyl)-4-methoxyethoxymethoxyphényl)-1-propényl] benzoïque.
Acide 4-[(Z)-2-(3-(1-adamantyl)-4-methoxyethoxymethoxyphényl)-1-propényl] benzoïque.
5-{2-[3-Adamantan-1-yl-4-(2-méthoxy-éthoxyméthoxy)-phényl]-propèn-(E)-yl}-pyridine-2-carboxylate de méthyle.
Acide 5-{2-[3-adamantan-1-yl-4-(2-méthoxy-éthoxyméthoxy)-phényl]-propèn-(E)-yl}-pyridine-2-carboxylique.
Acide 5-{2-[3-adamantan-1-yl-4-(2-méthoxy-éthoxyméthoxy)-phényl]-propèn-(Z)-yl}-pyridine-2-carboxylique.
6-{2-[3-Adamantan-1-yl-4-(2-méthoxy-éthoxyméthoxy)-phényl]-propèn-(E)-yl}-nicotinate d'éthyle.
Acide 6-{2-[3-adamantan-1-yl-4-(2-méthoxy-éthoxyméthoxy)-phényl]-propèn-(E)-yl}-nicotinique.
Acide 6-{2-[3-Adamantan-1-yl-4-(2-méthoxy-éthoxyméthoxy)-phényl]-propèn-(Z)-yl}-nicotinique
4-{2-[3-Adamantan-1-yl-4-(2-méthoxy-éthoxyméthoxy)-phényl]-propèn-(Z)-yl}-2-méthoxybenzoate de méthyle.
Acide 4-{2-[3-adamantan-1-yl-4-(2-méthoxy-éthoxyméthoxy)-phényl]-propèn-(E)-yl}-2-méthoxy-benzoique.
Acide 4-{2-[3-adamantan-1-yl-4-(2-méthoxy-éthoxyméthoxy)-phényl]-propèn-(Z)-yl}-2-méthoxy-benzoique.
4-{2-[3-adamantan-1-yl-4-(3-éthoxyméthoxy-propyl)-phényl]-propèn-(E/Z)-yl}-benzoate d'éthyle.
Acide 4-{2-[3-adamantan-1-yl-4-(3-éthoxyméthoxy-propyl)-phényl]-propèn-(E)-yl}-benzoïque.
Acide 4-{2-[3-adamantan-1-yl-4-(3-éthoxyméthoxy-propyl)-phényl]-propèn-(Z)-yl}-benzoïque.
4-{2-[3-adamantan-1-yl-4-(3-benzyloxy-propyl)-phényl]-propèn-(E/Z)-yl}-benzoate d'éthyle
Acide 4-{2-[3-adamantan-1-yl-4-(3-benzyloxy-propyl)-phényl]-propèn-(E)-yl}-benzoïque.
Acide 4-{2-[3-adamantan-1-yl-4-(3-benzyloxy-propyl)-phényl]-propèn-(Z)-yl}-benzoïque.
4-{2-[3-Adamantan-1-yl-4-(3-diethylcarbamoylmethoxy-propyl)-phenyl]-propenyl}-benzoate d'éthyle.
Acide 4-{2-[3-Adamantan-1-yl-4-(3-diethylcarbamoylmethoxy-propyl)-phenyl]-propenyl}-benzoïque.
4-{2-[3-adamantan-1-yl-4-(3-carboxymethoxy-propyl)-phenyl]-propenyl}-benzoate d'éthyle.
Acide 4-{2-[3-adamantan-1-yl-4-(3-carboxymethoxy-propyl)-phenyl]-propenyl}-benzoïque.
4-{2-[3-adamantan-1-yl-4-(3-carbamoylmethoxy-propyl)-phenyl]-propenyl}-benzoate d'éthyle.
Acide 4-{2-[3-adamantan-1-yl-4-(3-carbamoylmethoxy-propyl)phenyl] propenyl} benzoïque.
N-éthyl- 4-[(E)-2-(3-(1-adamantyl)-4-methoxyethoxymethoxyphényl)-1-propényl] benzamide.
4-[(E)-2-(3-(1-adamantyl)-4-methoxyethoxymethoxyphényl)-1-propényl] benzamide.
N-4-(hydroxyphenyl)-4-[(E)-2-(3-(1-adamantyl)-4-methoxyethoxymethoxyphényl)-1-propényl] benzamide.
4-[(E)-2-(3-(1-adamantyl)-4-methoxyethoxymethoxyphényl)-1-propényl] benzèneméthanol.
4-[(E)-2-(3-(1-adamantyl)-4-methoxyethoxymethoxyphényl)-1-propényl] benzaldéhyde.
4-[(E)-2-(3-(1-adamantyl)-4-methoxyethoxymethoxyphényl)-1-propényl]phénol.
Morpholide de l'acide 4-[(E)-2-(3-(1-adamantyl)-4-methoxyethoxymethoxyphényl)-1-propényl] benzoïque.
Acide 4-[(E)-2-(3-(1-adamantyl)-4-methoxyethoxymethylsulfanylphényl)-1-propényl] benzoïque.

Selon la présente invention, les composés de formule (I) plus particulièrement préférés sont ceux pour lesquels l'une au moins, et de préférence toutes, les conditions ci-dessous sont respectées :
- R₁ est le radical -CO-R₇
- Ar représente les radicaux de formule (a) ou (b),
- X et Y, identiques ou différents, représentent des atomes d'oxygène ou de soufre,
- R₃ représente un radical alkyle inférieur.

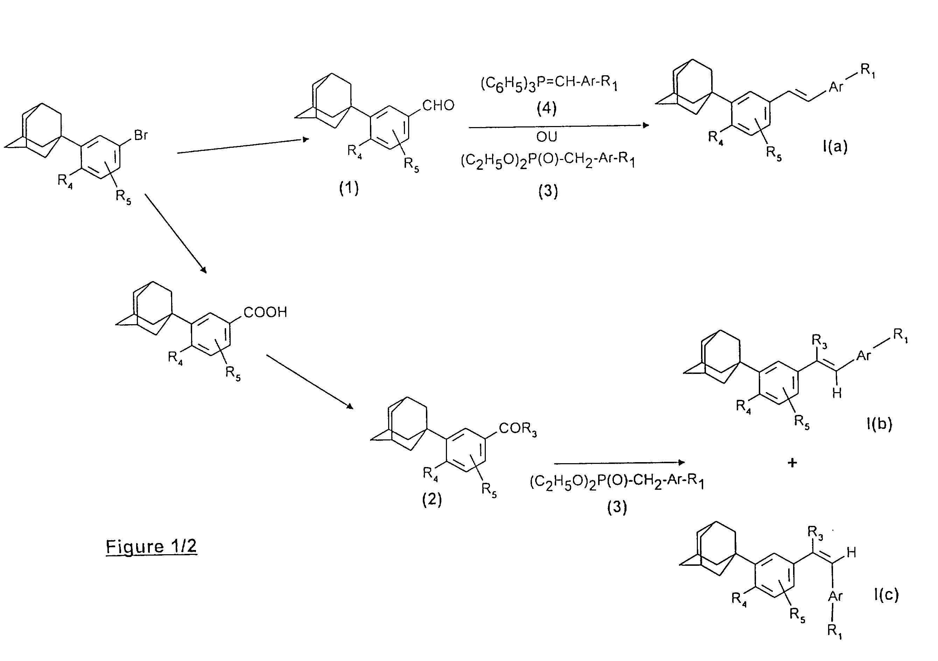

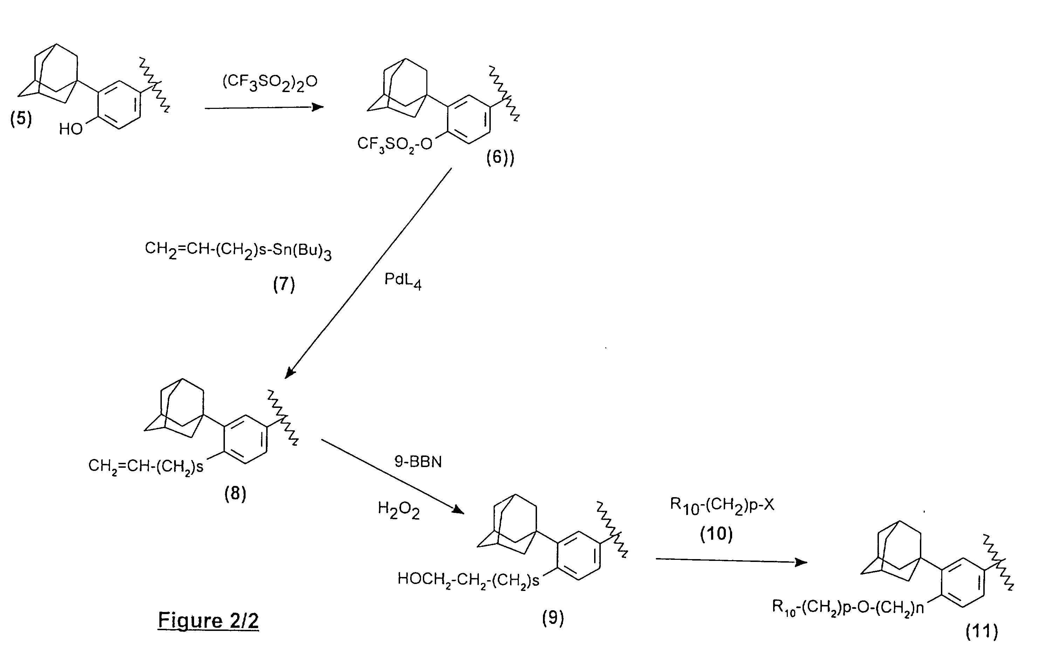

La présente invention a également pour objet les procédés de préparation des composés de formule (I), en particulier selon les schémas réactionnels donnés aux figures 1 et 2.

Ainsi les composés de formule I(a), I(b) et I(c) peuvent être obtenus (figure 1) en faisant réagir en milieu anhydre dans un solvant organique, de préférence le THF, un dérivé aldéhyde aromatiques (1) (lorsque R₃ est un atome d'hydrogène) ou un dérivé cétone aromatique (2) (lorsque R₃ est un radical alkyle inférieur) suivant une réaction de type Horner-Emmons ou Wittig avec des dérivés phosphonates aromatiques (3) ou des dérivés phosphines aromatiques (4) en présence d'hydrure de sodium ou de tert-butylate de potassium.
Dans ces réactions d'oléfination, l'isomère géométrique de configuration E peut aussi être obtenu par conversion sous irradiation sous lumière UV de l'isomère de configuration Z.
Lorsque R₄ représente les radicaux -(CH2)n-Y-(CH2)p-R₁₀ les composés peuvent être obtenus (figure 2) à partir des dérivés phénoliques (5) que l'on transforme en dérivés triflates (6), puis réaction avec un dérivé stannique (7) en présence d'un catalyseur au palladium (par exemple le chlorure de bis(triphénylphosphine)palladium(II) selon M. Echavarren et J.K. Stille J. Am. Chem. Soc 1987, 109, 5478-86. Les dérivés alcooliques (9) sont obtenus par réaction des dérivés éthyléniques (8) avec du 9-borabicyclo(3,3,1)nonane, puis oxydation avec l'eau oxygénée selon Knights, E. F et al J; Am. Chem. Soc. 1968, 90, 5281. Par alkylation avec un dérivé halogéné (10), on obtient les dérivés (11). Lorsque R₁ représente le radical -COOH, les composés sont préparés en protégeant R₁ par un groupe protecteur de type alkyle, allylique ou tert-butylique. Le passage à la forme libre peut être éffectué:
- dans le cas d'un groupe protecteur alkyle, au moyen de soude ou d'hydroxyde de lithium dans un solvant alcoolique, tel que le méthanol ou dans le THF.
- dans le cas d'un groupe protecteur allylique, au moyen d'un catalyseur, tel que certains complexes de métaux de transition en présence d'une amine secondaire, telle que la morpholine.
- dans le cas d'un groupement protecteur de type tert-butylique au moyen d'iodure de triméthylsilane.
Lorsque R₁ est un radical alcool, les composés peuvent être obtenus à partir de l'acide par réduction en présence d'hydrure double de lithium et d'aluminium.
Lorsque R₁ est un radical aldéhyde, les composés peuvent être obtenus à partir de l'alcool par oxydation avec de l'oxyde de manganèse ou du pyridinium dichromate.
Lorsque R₁ est un radical de type amide, les composés peuvent être préparés par transformation de l'acide en chlorure d'acide par exemple avec du chlorure de thionyle puis réaction avec l'ammoniaque ou une amine appropriée.

Les produits de formule générale (I) peuvent servir de produits de départ pour la fabrication d'autres composés de formule (I) selon l'invention. Ces composés sont obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles du groupe R₁ comme indiqué ci-dessous:
acide carboxylique → ester
ester → acide carboxylique
acide → chlorure d' acide
chlorure d' acide → amide
acide → amide
acide → alcool
alcool → aldéhyde
amide → amine
thiol → thioéther
thioéther → sulfoxyde
thioéther → sulfone
acide sulfonique → ester sulfonique
acide sulfonique → sulfonamide
acide suifinique → ester sulfinique

Les composés selon l'invention présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p. 793-801, 1978). Ces tests montrent les activités de ces composés respectivement dans les domaines de la différenciation et de la prolifération cellulaire. Dans le test de différenciation des cellules (F9), il est possible d'évaluer une activité agoniste, comme une activité antagoniste aux récepteurs de l'acide rétinoïque. En effet, un antagoniste est inactif lorsqu'il est seul dans ce test, mais inhibe partiellement ou totalement l'effet produit par un rétinoïde agoniste sur la morphologie et sur la sécrétion de l'activateur plasminogène. Certains de ces composés présentent donc également une activité dans un test qui consiste à identifier des molécules antagonistes des RARs, tel que décrit dans la demande de brevet français n° 95-07302 déposée le 19 juin 1995 par la Demanderesse. Ce test comprend les étapes suivantes : (i) on applique topiquement sur une partie de la peau d'un mammifère une quantité suffisante d'une molécule agoniste des RARs, (ii) on administre par voie systémique ou topique sur ce même mammifère ou sur cette même partie de la peau du mammifère, avant, pendant ou après l'étape (i), une molécule susceptible de présenter une activité antagoniste des RARs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être inhibée par l'administration par voie systémique ou topique d'une molécule antagoniste des RARs. En outre, certains de ces composés peuvent apporter une synergie à l'activité biologique de produits se liant aux récepteurs nucléaires.

La présente invention a également pour objet à titre de médicament les composés de formule (I) telle que définie ci-dessus.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané, telle que le syndrome de Kaposis, ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose ou l'hypertension ainsi que le diabète non-insulino dépendant,
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétokonazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### A. EXEMPLES DE COMPOSES

### EXEMPLE 1

### 4-[(E)-2-(3-(1-adamantyl)-4-méthoxyéthoxyméthoxyphényl)éthényl]benzoate d'éthyle

### (a) 3-(1-adamantyl)-1-bromo-4-méthoxyéthoxyméthoxybenzène.

Dans un tricol et sous courant d'azote, on introduit 3,8 g (0,13 mole) d'hydrure de sodium (80% dans l'huile), 50 ml de DMF, ajoute goutte à goutte une solution de 40,0 g (0,13 mole) de 2-(1-adamantyl)-4-bromophénol en solution dans 100 ml de DMF et agite jusqu'a cessation du dégagement gazeux. On ajoute ensuite goutte à goutte une solution de 18 ml (0,15 mole) de chlorure de 2-méthoxyéthoxyméthyle dans 20 ml de DMF et agite pendant quatre heures à la température ambiante. On verse le milieu réactionnel dans l'eau, extrait avec de l'éther éthylique, décante la phase organique, lave à l'eau, sèche sur sulfate de magnésium, évapore. Le résidu est purifié par chromatographie sur colonne de silice élué avec un mélange de dichlorométhane et d'hexane (50:50). Après évaporation des solvants, on recueille 40,1 g (78%) du produit attendu de point de fusion 69-70°C.

### (b) 3-(1-adamantyl)-4-méthoxyéthoxyméthoxyphénylcarboxaldéhyde.

Dans un tricol et sous courant d'azote, on introduit 34,0 g (89,0 mmoles) de 3-(1-adamantyl)-1-bromo-4-méthoxyéthoxyméthoxybenzène et 250 ml de THF. A -78°C on ajoute goutte à goutte 43 ml (106 mmoles) d'une solution de n-butyllithium (2,5 M dans l'hexane) et agite 30 minutes. On ajoute ensuite goutte à goutte 8,3 ml (106 mmoles) de DMF et laisse remonter à température ambiante. On verse le milieu réactionnel dans une solution aqueuse de chlorure d'ammonium, extrait avec de l'éther éthylique, décante la phase organique, sèche sur sulfate de magnésium, évapore. Le résidu obtenu est purifié par chromatographie sur colonne de silice élué avec du dichlorométhane. Après évaporation des solvants, on recueille 17,6 g (58%) d'aldéhyde attendu de point de fusion 63-64°C.

### (c) 4-[(E)-2-(3-(1-adamantyl)-4-méthoxyéthoxyméthoxyphényl)éthényl]benzoate d'éthyle.

Dans un tricol et sous courant d'azote, on introduit 360 mg (12 mmoles) d'hydrure de sodium (80% dans l'huile) et 20 ml de THF. On ajoute goutte à goutte une solution de 70 ml de THF contenant 3,44 g (10 mmoles) de 3-(1-adamantyl)-4-méthoxyéthoxyméthoxyphénylcarboxaldéhyde, 3,6 g (12 mmoles) de 4-éthoxycarbonylbenzylphosphonate de diéthyle et 440 mg d'éther couronne (15-crown-5). On agite à température ambiante pendant quatre heures, verse le milieu réactionnel dans l'eau, extrait avec de l'éther éthylique, décante la phase organique, sèche sur sulfate de magnésium, évapore. Le résidu obtenu est trituré dans l'hexane, filtré et séché. On recueille 4,55 g (93%) de l'ester éthylique attendu de point de fusion 87-88°C.

### EXEMPLE 2

### Acide4-[(E)-2-(3-(1-adamantyl)-4-méthoxyéthoxyméthoxyphényl)éthényl] benzoïque.

Dans un ballon, on introduit 2,0 g (4,0 mmoles) de 4-[(E)-2-[3-(1-adamantyl)-4-méthoxyéthoxyméthoxyphényl)éthényl]benzoate d'éthyle et une solution de soude méthanolique (1,6 g d'hydroxyde de sodium dans 50 ml de méthanol). On chauffe à reflux pendant quatre heures, évapore à sec, reprend par l'eau, acidifie à pH 1. On extrait la phase aqueuse avec de l'acétate d'éthyle, décante la phase organique, sèche sur sulfate de magnésium, évapore. Le résidu obtenu est trituré dans le minimum d'éther éthylique, filtré, séché. On recueille 1,4 g (74%) de l'acide attendu de point de fusion 207-208°C.

### EXEMPLE 3

### 4-[(E)-2-(3-(1-adamantyl)-4-méthoxyéthoxyméthoxyphényl)-1-propényl] benzoate d'éthyle.

### (a) acide 3-(1-adamantyl)-4-méthoxyéthoxyméthoxybenzoïque.

Le 3-(1-adamantyl)-1-bromo-4-méthoxyéthoxyméthoxybenzène (28,5 g, 72 mmoles) est dissous dans 200 ml de THF. La solution obtenue est ajoutée goutte à goutte sur du magnésium (2,4 g, 100 mmoles) et un cristal d'iode. Après introduction, on chauffe à reflux pendant deux heures, refroidit à -78°C et fait passer un courant de CO₂ pendant une heure. On laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est trituré dans l'hexane, filtré et séché. On recueille 15,5 g (60%) de l'acide attendu de point de fusion 115-116°C.

### (b) 3-(1-adamantyl)-4-méthoxyéthoxyméthoxyacétophenone.

Dans un tricol et sous courant d'azote on introduit 15,5 g (43 mmoles) d'acide 3-(1-adamantyl)-4-méthoxyéthoxyméthoxybenzoïque et 300 ml d'éther éthylique anhydre. A -20°C, on ajoute goutte à goutte 80 ml (0,13 mole) de méthyllithium (1,6 M dans l'éther), puis agite pendant trois heures à la température ambiante. On verse le milieu réactionnel dans une solution aqueuse saturée en chlorure d'ammonium, décante la phase organique, sèche sur sulfate de magnésium, évapore. On recueille 15,4 g (100%) de l'acétophénone attendu sous forme d'une huile légèrement jaune.

### (c) 4-[(E)-2-(3-(1-adamantyl)-4-méthoxyéthoxyméthoxyphényl)-1-propényl]benzoate d'éthyle.

De manière analogue à l'exemple 1(c), par réaction de 3,58 g (10 mmoles) de 3-(1-adamantyl)-4-méthoxyéthoxyméthoxyacétophenone avec 3,13 g (12 mmoles) de 4-éthoxycarbonylbenzylphosphonate de diéthyle, on obtient après chromatographie sur colonne de silice élué avec un mélange d'heptane et d'acétate d'éthyle (90:10), 2,5 g (50%) d'ester éthylique sous forme d'un mélange d'isomères (E) et (Z).

### EXEMPLE 4

### Acide 4-[(E)-2-(3-(1-adamantyl)-4-méthoxyéthoxyméthoxyphényl)-1-propényl] benzoïque.

De manière analogue à l'exemple 2 à partir de 2,5 g (5 mmoles) d'un mélange (E) et (Z) de 4-[2-(3-(1-adamantyl)-4-méthoxyéthoxyméthoxyphényl)-1-propényl] benzoate d'éthyle et après recristallisation dans l'éthanol, on recueille 150 mg (13%) d'acide 4-[(E)-2-(3-(1-adamantyl)-4-méthoxyéthoxyméthoxyphényl)-1-propényl] benzoïque de point de fusion 177-178°C.

### EXEMPLE 5

### Acide 4-[(Z)-2-(3-(1-adamantyl)-4-méthoxyéthoxyméthoxyphényl)-1-propényl] benzoïque.

Le filtrat de recristallisation obtenu à l'exemple 4 est évaporé à sec. Le solide obtenu est recristallisé dans l'éthanol et l'on recueille ainsi, après filtration, 140 mg (12%) de l'acide 4-[(Z)-2-(3-(1-adamantyl)-4-méthoxyéthoxyméthoxyphényl)-1-propényl] benzoïque de point de fusion 198-199°C.

### EXEMPLE 6

### 5-{2-[3-Adamantan-1-yl-4-(2-méthoxy-éthoxyméthoxy)-phényl]-propèn-(E)-yl}-pyridine-2-carboxylate de méthyle.

### (a) 2-bromo-5-méthyl-pyridine.

Dans un réacteur de deux litres, on mélange 500 ml d'acide bromhydrique aqueux (47%) avec 50,0 g (462 mmoles) de 2-amino-5-méthyl pyridine. La solution obtenue est refroidie à -20°C, puis on coule en dix minutes 206,9 g (1,3 mole) de brome, en maintenant la température comprise entre -15°C et -25°C. Le milieu réactionnel est agité pendant une heure à -20°C, puis on coule goutte à goutte une solution composée de 82,7 g (1,2 mole) de nitrite de sodium en solution dans 300 ml d'eau, en vingt minutes.
Le milieu réactionnel est agité pendant quatre heures à la température ambiante, puis on refroidit à -10°C, ajoute 800 ml d'une solution de soude aqueuse (pH 10), agite cinq minutes, extrait avec de l'éther éthylique, décante la phase organique, lave à l'eau puis à l'aide d'une solution de Na₂S₂O₃, sèche sur sulfate de magnésium, évapore. On recueille 75,2 g (94%) du composé attendu sous la forme de cristaux orangés de point de fusion 38-42°C.

### (b) 2-bromo-5-bromométhyl-pyridine.

Dans un tricol de 250 ml, on mélange 10,0 g (58,0 mmoles) du composé bromé obtenu à l'exemple 4(a) et 70 ml de tétrachlorure de carbone. On chauffe à environ 35°C et introduit en une fois un mélange broyé composé de 10,32 g (58,0 mmoles) de N-bromosuccinimide et de 421 mg (1,74 mmole) de peroxyde de benzoyle. Le milieu réactionnel est chauffé à reflux et sous irradiation lumineuse (1000 W) pendant trois heures. Le mélange est refroidi, filtré, le filtrat évaporé à sec, repris par du dichlorométhane, lavé à l'aide d'une solution saturée de bicarbonate de sodium, puis la phase organique est séchée sur sulfate de magnésium et évaporée. Le résidu obtenu est purifié par chromatographie sur colonne de silice élué avec un mélange composé de dichlorométhane et d'heptane (1:1). Après évaporation des solvants, on recueille 2,9 g (20%) du composé attendu sous la forme de cristaux beiges.

### (c) (6-bromo-pyridin-3-ylméthyl)-phosphonate de diéthyle.

Dans un ballon de 100 ml, on mélange 3,0 g (11,9 mmoles) du composé obtenu à l'exemple 6(b) et 10 ml de triéthylphosphite.
Le mélange est chauffé à reflux pendant trente minutes, refroidi et évaporé à sec. Le résidu obtenu est purifié par chromatographie sur colonne de silice élué avec un mélange d'acétate d'éthyle et d'heptane (6:4). Après évaporation des solvants, on recueille 3,12 g (80%) du composé attendu sous la forme d'une huile jaune claire.

### (d) 5-{2-[3-adamantan-1-yl-4-(2-méthoxy-éthoxyméthoxy)-phényl]-propèn-(E)-yl}-2-bromo-pyridine.

Dans un ballon de 100 ml, on mélange 2,6 g (8,4 mmoles) du composé obtenu à l'exemple 6(c), 5 ml de DMPU et 5 ml de THF. On ajoute en une fois 250 mg (8,4 mmoles) d'hydrure de sodium à 80%, agite à la température ambiante pendant trente minutes, puis à 40°C pendant quinze minutes. On ajoute goutte à goutte une solution de 2,5 g (7,0 mmoles) de la cétone obtenue à l'exemple 3(b) en solution dans 10 ml de THF. Le milieu réactionnel est agité pendant quatre heures à la température ambiante, puis on ajoute une solution saturée d'acide citrique, extrait avec de l'éther éthylique, décante la phase organique, lave à l'eau, sèche sur sulfate de magnésium, évapore. Le résidu obtenu est purifié par chromatographie sur colonne de silice élué avec un mélange d'acétate d'éthyle et d'heptane (2:8). On recueille 2,69 g (75%) du composé attendu sous la forme d'une huile jaune.

### (e) 5-{2-[3-adamantan-1-yl-4-(2-méthoxy-éthoxyméthoxy)-phényl]-propèn-(E)/(Z)-yl}-pyridine-2-carboxylate de méthyle.

Dans une bombe à hydrogéner, on introduit 4,1 g (8,0 mmoles) du composé bromé obtenu à l'exemple 6(d), 890 mg (1,6 mmole) de 1,1'-bis(diphénylphosphino) ferrocène, 180 mg (0,8 mmole) d'acétate de palladium, 20 ml de DMF, 2,23 ml (16,0 mmoles) de triéthylamine et 3,24 ml (80 mmoles) de méthanol. Le milieu réactionnel est confiné sous une pression de trois bars de monoxyde de carbone et chauffé à 100°C pendant trois heures. Le mélange est refroidi, repris par de l'eau, extrait par de l'acétate d'éthyle, lavé à l'eau, puis la phase organique est séchée sur sulfate de magnésium et évaporée. Le résidu obtenu est purifié par chromatographie sur colonne de silice élué avec un mélange d'acétate d'éthyle et d'heptane (2:8). Après évaporation des solvants, on recueille 1,3 g (33%) de l'isomère (Z) sous la forme d'une huile jaune, et 1,16 g (29%) de l'isomère (E) sous la forme d'une poudre jaune claire de point de fusion 96-102°C.

### EXEMPLE 7

### Acide 5-{2-[3-adamantan-1-yl-4-(2-méthoxy-éthoxyméthoxy)-phényl]-propèn-(E)-yl}-pyridine-2-carboxylique.

De manière analogue à l'exemple 2 à partir de 1,16 g (2,36 mmoles) de l'isomère (E) du 5-{2-[3-adamantan-1-yl-4-(2-méthoxy-éthoxyméthoxy)-phényl]-propèn-yl}-pyridine-2-carboxylate de méthyle obtenu à l'exemple 6(e) et après purification par trituration dans un mélange d'éther éthylique et d'heptane (5:5), on recueille 0,98 g (86%) d'acide 5-{2-[3-adamantan-1-yl-4-(2-méthoxyéthoxyméthoxy)-phényl]-propèn-(E)-yl}-pyridine-2-carboxylique sous la forme d'une poudre beige de point de fusion 91-95°C.

### EXEMPLE 8

### Acide 5-{2-[3-adamantan-1-yl-4-(2-méthoxy-éthoxyméthoxy)-phényl]-propén-(Z)-yl}-pyridine-2-carboxylique.

De manière analogue à l'exemple 2 à partir de 1,30 g (2,64 mmoles) de l'isomère (Z) du 5-{2-[3-adamantan-1-yl-4-(2-méthoxy-éthoxyméthoxy)-phényl]-propèn-yl}-pyridine-2-carboxylate de méthyle obtenu à l'exemple 6(e), et après trituration dans l'heptane, on recueille 1,05 g (83%) d'acide 5-{2-[3-adamantan-1-yl-4-(2-méthoxy-éthoxyméthoxy)-phényl]-propèn-(Z)-yl}-pyridine-2-carboxylique sous la forme d'une poudre blanche de point de fusion 91-95°C.

### EXEMPLE 9

### 6-{2-[3-Adamantan-1-yl-4-(2-méthoxy-éthoxyméthoxy)-phényl]-propèn-(E)-yl}-nicotinate d'éthyle.

### (a) 6-hydroxyméthyl-nicotinate d'éthyle.

Dans un ballon de un litre, on dissous 45,77 g (205 mmoles) de 2,5 pyridine dicarboxylate d'éthyle dans 410 ml d'éthanol absolu. On refroidit le milieu réactionnel à -5°C et ajoute 5,04 g (133,2 mmoles) de borohydrure de sodium, puis, par petites fractions, 14,79 g (133,2 mmoles) de chlorure de calcium, en maintenant la température inférieure à -5°C. Le milieu réactionnel est agité à -5°C pendant deux heures, versé dans l'eau, le produit extrait par de l'éther éthylique, lavé à l'eau, puis la phase organique est séchée sur sulfate de magnésium et évaporée. Le résidu obtenu est purifié par chromatographie sur colonne de silice élué par de l'éther éthylique, pour obtenir 31,33 g (84%) d'un solide blanc cristallisé.

### (b) 6-bromométhyl-nicotinate d'éthyle.

Dans un ballon de 500 ml, on dissous 10,0 g (55,2 mmoles) du composé obtenu à l'exemple 9(a), 36,6 g (110 mmoles) de tétrabromure de carbone et 100 ml d'éther éthylique. Le milieu réactionnel est refroidi à 0°C, puis on ajoute en dix minutes 40,78 g (110 mmoles) de trioctylphosphine. Le milieu réactionnel est agité pendant quinze minutes à la température ambiante, versé dans l'eau, le produit extrait par de l'éther éthylique, lavé à l'eau, puis la phase organique est séchée sur sulfate de magnésium et évaporée. Le résidu obtenu est purifié par chromatographie sur colonne de silice élué par de l'éther éthylique, pour obtenir 11,70 g (87%) d'une huile rouge.

### (c) 6-(diéthoxy-phosphoryl)-nicotinate d'éthyle.

Dans un ballon de 100 ml, on mélange 11,0 g (45,0 mmoles) du composé obtenu à l'exemple 9(b) et 100 ml de triéthyle phosphite. Le mélange est chauffé à reflux pendant cinq minutes, refroidi et évaporé à sec. Le résidu obtenu est purifié par chromatographie sur colonne de silice élué avec un mélange d'acétate d'éthyle et d'heptane (8:2). Après évaporation des solvants, on recueille 9,7 g (71%) du composé attendu sous la forme d'une huile jaune.

### (d) 6-{2-[3-adamantan-1-yl-4-(2-méthoxy-éthoxyméthoxy)-phényl]-propèn-(E)-yl}-nicotinate d'éthyle.

Dans un ballon de 50 ml, on mélange 0,5 g (1,66 mmoles) du composé obtenu à l'exemple 9(c), 5 ml de DMPU et 5 ml de THF. On ajoute en une fois 50 mg (1,66 mmoles) d'hydrure de sodium à 80%, agite à la température ambiante pendant quinze minutes puis à 40°C pendant quinze minutes supplémentaires. On ajoute goutte à goutte une solution de 496 mg (1,4 mmoles) de la cétone obtenue à l'exemple 3(b) en solution dans 5 ml de THF. Le milieu réactionnel est agité pendant quatre heures à la température ambiante, puis on ajoute une solution saturée d'acide citrique, extrait avec de l'éther éthylique, décante la phase organique, lave à l'eau, sèche sur sulfate de magnésium, évapore. Le résidu obtenu est purifié par chromatographie sur colonne de silice élué avec un mélange d'acétate d'éthyle et d'heptane (2:8). On recueille 60 mg (7%) du composé (E) attendu, sous la forme d'un solide jaune de point de fusion 78°C, ainsi qu'une fraction correspondant au composé (Z), qui sera mis directement en réaction pour l'obtention de l'acide correspondant (exemple 11).

### EXEMPLE 10

### Acide 6-{2-[3-adamantan-1-yl-4-(2-méthoxy-éthoxyméthoxy)-phényl]-propèn-(E)-yl}-nicotinique.

De manière analogue à l'exemple 2 à partir de 1,30 g (2,57 mmoles) de l'isomère (E) du 6-{2-[3-adamantan-1-yl-4-(2-méthoxy-éthoxyméthoxy)-phényl]-propèn-yl}-nicotinate d'éthyle obtenu à l'exemple 9(d), et après purification par chromatographie sur colonne de silice élué avec un mélange d'acétate d'éthyle et d'heptane (5:5), on recueille 0,98 g (80%) du composé attendu sous la forme d'une poudre blanc cassé de point de fusion 86-90°C.

### EXEMPLE 11

### Acide 6-{2-[3-Adamantan-1-yl-4-(2-méthoxy-éthoxyméthoxy)-phényl]-propèn-(Z)-yl}-nicotinique.

De manière analogue à l'exemple 2 à partir de 463 mg (0,92 mmoles) de l'isomère (Z) du 6-{2-[3-adamantan-1-yl-4-(2-méthoxy-éthoxyméthoxy)-phényl]-propèn-yl}-nicotinate d'éthyle obtenu à l'exemple 9(d), et après cristallisation dans un mélange d'éther éthylique et d'heptane (5:5), on recueille 350 mg (80%) du composé attendu sous la forme d'une poudre blanc cassé de point de fusion 191-195°C.

### EXEMPLE 12

### 4-{2-[3-Adamantan-1-yl-4-(2-méthoxy-éthoxyméthoxy)-phényl]-propèn-(Z)-yl}-2-méthoxybenzoate de méthyle.

De manière analogue à l'exemple 6(d) à partir de 13,96 g (50,9 mmoles) de 4-méthoxycarbonylbenzylphosphonate de diéthyle et 9,13 g (25,45 mmoles) de la cétone obtenue à l'exemple 3(b), on recueille 8,25 g (62%) du composé (Z) sous la forme d'un solide blanc cristallisé et 3,00 g (23%) du composé (Z) sous la forme d'une huile incolore.

### EXEMPLE 13

### Acide 4-{2-[3-adamantan-1-yl-4-(2-méthoxy-éthoxyméthoxy)-phényl]-propèn-(E)-yl}-2-méthoxy-benzoique.

De manière analogue à l'exemple 2 à partir de 3,00 g (5,76 mmoles) de l'isomère (E) du 4-{2-[3-adamantan-1-yl-4-(2-méthoxy-éthoxyméthoxy)-phényl]-propèn-yl}-2-méthoxy-benzoate de méthyle obtenu à l'exemple 12, et après cristallisation dans le méthanol, on recueille 2,80 g (96%) du composé attendu sous la forme de cristaux jaunes.

### EXEMPLE 14

### Acide 4-{2-[3-adamantan-1-yl-4-(2-méthoxy-éthoxyméthoxy)-phényl]-propèn-(Z)-yl}-2-méthoxy-benzoique.

De manière analogue à l'exemple 2 à partir de 7,60 g (14,6 mmoles) de l'isomère (Z) du 4-{2-[3-adamantan-1-yl-4-(2-méthoxy-éthoxyméthoxy)-phényl]-propèn-yl}-2-méthoxy-benzoate de méthyle obtenu à l'exemple 12, et après cristallisation dans un mélange d'éther éthylique et d'hexane (10:90), on recueille 7,39 g (100%) du composé attendu sous la forme de cristaux blancs de point de fusion 113°C.

### EXEMPLE 15

### 4-{2-[3-adamantan-1-yl-4-(3-éthoxyméthoxy-propyl)-phényl]-propèn-(E/Z)-yl}-benzoate d'éthyle.

### (a) 3-adamantan-1-yl-4-trifluorométhanesulfonyloxy-benzoate de méthyle.

Dans un tricol de 1 I, sous atmosphère d'azote, on mélange 28,6 g (100,0 mmoles) de 3-(1-adamantyl)-4-hydroxy benzoate de méthyle avec 122 mg (1,0 mmole) de N,N-diméthylaminopyridine, 200 ml de dichlorométhane et 28 ml de pyridine. Le mélange est refroidi à -78°C et on ajoute goutte à goutte 20,19 ml (120,0 mmoles) d'anhydride triflique. La température est remontée à la température ambiante, et le mélange maintenu deux heures sous agitation. Le milieu réactionnel est versé dans une solution d'acide chlorhydrique 1N, extrait par de l'éther éthylique, lavé à l'eau, séché sur sulfate de magnésium, filtré et évaporé à sec. On recueille 41,5 g (99%) du composé attendu sous la forme d'une poudre orange claire de point de fusion 95°C.

### (b) 3-adamantan-1-yl-4-allyl-benzoate de méthyle.

Dans un tricol de 1 I, sous atmosphère d'azote, on mélange 23,06 g (55,1 mmoles) du composé obtenu à l'exemple 15(a) avec 4,66 g (110,0 mmole) de chlorure de lithium, 5,8 g (8,3 mmoles) de chlorure de bistriphénylphosphine palladium(ll), 21,89 g (66,0 mmoles) d'allyltributylétain et 300 ml de N,N-diméthylformamide. Le mélange est chauffé à 100°C pendant deux heures puis versé dans une solution d'acide chlorhydrique 1N, extrait par de l'éther éthylique, lavé à l'eau, séché sur sulfate de magnésium, filtré et évaporé à sec. Le résidu obtenu est purifié par distillation des sels d'étain (108-116°C sous 8.10⁻² bar), puis par chromatographie sur colonne de silice élué par de l'heptane. Après évaporation des solvants, on recueille 12,75 g (74%) du composé attendu sous la forme d'une huile jaune.

### (c) 3-adamantan-1-yl-4-(3-hydroxy-propyl)-benzoate de méthyle.

Dans un tricol de 250 ml, sous atmosphère d'azote, on solubilise 12,40 g (40,0 mmoles) du composé obtenu à l'exemple 15(b) dans 250 ml de tétrahydrofuranne. Le mélange est refroidi à 0°C, puis on coule goutte à goutte 240 ml (120,0 mmoles) de 9-borabicyclo-[3,3,1]-nonane (9-BBN) et agite à la température ambiante pendant deux heures. Le milieu réactionnel est refroidi de nouveau à 0°C puis on coule goutte à goutte 124 ml (124 mmoles) d'une solution aqueuse de soude (1M), puis, toujours à 0°C, 102 ml (1 mole) d'une solution d'eau oxygénée à 30%. Le milieu réactionnel est agité pendant trente minutes à la température ambiante puis versé dans une solution d'acide chlorhydrique 1N, extrait par de l'éther éthylique, lavé à l'eau, séché sur sulfate de magnésium, filtré et évaporé à sec. Le résidu est purifié par chromatographie sur colonne de silice élué par un mélange composé de 30% d'acétate d'éthyle et 70% d'heptane. On recueille 9,4 g (71%) du composé attendu sous la forme d'une poudre blanche de point de fusion 77°C.

### (d) 3-adamantan-1-yl-4-(3-éthoxyméthoxy-propyl)-benzoate de méthyle.

Dans un tricol de 500 ml, sous atmosphère d'azote, on solubilise 8,00 g (24,3 mmoles) du composé obtenu à l'exemple 15(c) dans 200 ml de toluène. On ajoute 577 mg (1,7 mmole) d'hydrogénosulfate de tetrabutylamine, refroidit à 0°C, puis on coule goutte à goutte 6,78 ml (73,0 mmoles) de chlorure d'éthoxyméthyle et 120 ml de soude 10M aqueuse. Le milieu réactionnel est agité à 0°C pendant trente minutes puis versé dans une solution d'acide chlorhydrique 1N, extrait par de l'éther éthylique, lavé à l'eau, séché sur sulfate de magnésium, filtré et évaporé à sec. On recueille 9,39 g (100%) du composé attendu sous la forme d'une huile jaune claire.

### (e) acide 3-adamantan-1-yl-4-(3-éthoxyméthoxy-propyl)-benzoïque.

De manière analogue à l'exemple 2 à partir de 9,39 g (24,3 mmoles) du composé obtenu à l'exemple 15(d), on recueille 8,52 g (94%) du composé attendu sous la forme d'une poudre blanche de point de fusion 115°C.

### (f) 1-[3-adamantan-1-yl-4-(3-éthoxyméthoxy-propyl)-phényl]-éthanone.

De manière analogue à l'exemple 3(b) à partir de 8,00 g (21,5 mmoles) du composé obtenu à l'exemple 15(e), on recueille 5,34 g (67%) du composé attendu sous la forme d'une huile incolore.

### (g) 4-{2-[3-adamantan-1-yl-4-(3-éthoxyméthoxy-propyl)-phényl]-propèn-(E/Z)-yl}-benzoate d'éthyle.

De manière analogue à l'exemple 1(c), par réaction de 5,00 g (13,5 mmoles) du composé obtenu à l'exemple 15(f) avec 8,10 g (27,0 mmoles) de 4-éthoxycarbonylbenzylphosphonate de diéthyle, on obtient après chromatographie sur colonne de silice élué avec un mélange d'heptane et d'acétate d'éthyle (90:10), 6,3 g (90%) d'ester éthylique sous forme d'un mélange d'isomères (E) et (Z).

### EXEMPLE 16

### Acides 4-{2-[3-adamantan-1-yl-4-(3-éthoxyméthoxy-propyl)-phényl]-propèn-(E/Z)-yl}-benzoïques..

De manière analogue à l'exemple 2 à partir de 1,00 g (1,9 mmole) du mélange de composés (E) et (Z) obtenus à l'exemple 15(g), on recueille après chromatographie sur colonne de silice élué avec un mélange d'heptane et d'acétate d'éthyle (80:20), 330 mg (34%) du composé attendu (isomère Z) sous la forme d'une poudre blanche de point de fusion 161°C, et 70 mg (7%) du composé attendu (isomère E) sous la forme d'une poudre blanche de point de fusion 165°C

### EXEMPLE 17

### 4-{2-[3-adamantan-1-yl-4-(3-benzyloxy-propyl)-phényl]-propèn-(E/Z)-yl}-benzoate d'éthyle

### (a) 4-{2-[3-adamantan-1-yl-4-(3-hydroxy-propyl)-phényl]-propèn-(E/Z)-yl}-benzoate d'éthyle.

Dans un tricol de 250 ml, sous atmosphère d'azote, on solubilise 5,00 g (9,68 mmoles) du composé obtenu à l'exemple 15(g) dans 50 ml déthanol absolu. On ajoute goutte à goutte 5,18 ml (96,8 mmoles) d'acide sulfurique concentré. Le milieu réactionnel est agité à la température ambiante pendant 24 heures, puis pendant 5 heures à 40°C, versé dans l'eau, extrait par de l'éther éthylique, lavé à l'eau, séché sur sulfate de magnésium, filtré et évaporé à sec. On recueille 4,40 g (99%) du composé attendu sous la forme d'une huile jaune claire.

### (b) 4-{2-[3-adamantan-1-yl-4-(3-benzyloxy-propyl)-phényl]-propèn-(E/Z)-yl}-benzoate d'éthyle.

De manière analogue à l'exemple 15(d) à partir de 1,00 g (2,18 mmoles) du composé obtenu à l'exemple 17(a) et 0,78 ml (6,54 mmoles) de bromure de benzyle, on recueille 1,19 g (100%) du composé attendu sous la forme d'une huile jaune.

### EXEMPLE 18

### Acides 4-{2-[3-adamantan-1-yl-4-(3-benzyloxy-propyl)-phényl]-propèn-(E/Z)-yl}-benzoïques.

De manière analogue à l'exemple 2 à partir de 1,00 g (1,82 mmole) du mélange de composés (E) et (Z) obtenus à l'exemple 17(b), on recueille après chromatographie sur colonne de silice élué avec un mélange d'heptane et d'acétate d'éthyle (90:10), 480 mg (50%) du composé attendu (isomère Z) sous la forme d'une poudre blanche de point de fusion 166°C, et 175 mg (18%) du composé attendu (isomère E) sous la forme d'une poudre blanche de point de fusion 243°C.

### EXEMPLE 19

### 4-{2-[3-Adamantan-1-yl-4-(3-diethylcarbamoylmethoxy-propyl)-phenyl]-propenyl}-benzoate d'éthyle.

De manière analogue à l'exemple 15(d) à partir de 1,00 g (2,18 mmoles) du composé obtenu à l'exemple 17(a) et 0,83 ml (6,54 mmoles) de N,N-diéthylchloroacétamide, on recueille 1,00 g (80%) du composé attendu sous la forme d'une huile jaune claire.

### EXEMPLE 20

### Acide 4-{2-[3-Adamantan-1-yl-4-(3-diethylcarbamoylmethoxy-propyl)-phenyl]-propenyl}-benzoïque.

De manière analogue à l'exemple 2 à partir de 1,00 g (1,75 mmole) du mélange de composés (E) et (Z) obtenus à l'exemple 19(a), on recueille après chromatographie sur colonne de silice élué avec un mélange d'heptane et d'acétate d'éthyle (90:10), 480 mg (50%) du mélange (Z + E) des composés attendus.

### EXEMPLE 21

### 4-{2-[3-adamantan-1-yl-4-(3-carboxymethoxy-propyl)-phenyl]-propenyl}-benzoate d'éthyle.

### (a)4-{2-[3-Adamantan-1-yl-4-(3-tert.butoxycarbonylmethoxy-propyl)-phenyl]-propenyl}-benzoate d'éthyle

De manière analogue à l'exemple 15(d) à partir de 2,45 g (5,34 mmoles) du composé obtenu à l'exemple 17(a) et 2,59 ml (16,0 mmoles) de bromoacétate de ter.butyle, on recueille après chromatographie sur colonne de silice élué avec un mélange d'heptane et d'acétate d'éthyle (90:10), 2,65 g (86%) du mélange (Z + E) des composés attendus sous la forme d'une huile incolore.

### (b) 4-{2-[3-adamantan-1-yl-4-(3-carboxymethoxy-propyl)-phenyl]-propenyl}-benzoate d'éthyle.

Dans un ballon de 100 ml, sous atmosphère d'azote, on solubilise 2,60 g (4,54 mmoles) du composé obtenu à l'exemple 21(a) dans 45 ml de dichlorométhane et on ajoute goutte à goutte 3,50 ml (45,4 mmoles) d'acide trifluoroacétique. Le milieu réactionnel est agité à la température ambiante pendant 16 heures, puis pendant 8 heures au reflux, versé dans l'eau, extrait par du dichlorométhane, lavé à l'eau, séché sur sulfate de magnésium, filtré et évaporé à sec. On recueille 2,35 g (100%) du composé attendu sous la forme d'une huile jaune claire.

### EXEMPLE 22

### Acides 4-{2-[3-adamantan-1-yl-4-(3-carboxymethoxy-propyl)-phenyl]-propenyl}-benzoïques.

De manière analogue à l'exemple 2 à partir de 300 mg (0,60 mmole) du mélange de composés (E) et (Z) obtenus à l'exemple 21(b), on recueille après trituration dans un mélange composé de 20% d'éther éthylique et 80% d'heptane, 150 mg (51%) du mélange (Z + E) des composés attendus.

### EXEMPLE 23

### 4-{2-[3-adamantan-1-yl-4-(3-carbamoylmethoxy-propyl)-phenyl]-propenyl}-benzoate d'éthyle.

Dans un ballon de 100 ml, sous atmosphère d'azote, on solubilise 1,00 g (1,93 mmole) du composé obtenu à l'exemple 21(b) dans 20 ml de dichlorométhane et on ajoute goutte à goutte 0,42 ml (2,13 mmoles) de dicyclohexylamine. Le milieu réactionnel est agité à la température ambiante pendant cinq minutes, puis on ajoute goutte à goutte 0,14 ml (1,93 mmole) de chlorure de thionyle et on agite à la température ambiante pendant vingt minutes. Le milieu réactionnel est évaporé à sec, repris par de l'éther éthylique, filtré, on évapore le filtrat pour obtenir une huile jaune que l'on reprend dans 10 ml de THF pour obtenir une solution. Cette dernière est ajoutée goutte à goutte sur une solution composée de 0,13 ml d'une solution aqueuse à 32% d'ammoniaque (2,13 mmoles) dans 20 ml de THF, et 0,32 ml (2,32 mmoles) de triéthylamine. Le milieu réactionnel est agité à la température ambiante pendant cinq minutes, versé dans l'eau, et le pH est ajusté à 2 par addition d'HCl 0,5N. Le produit est extrait par de l'éther éthylique, lavé à l'eau, séché sur sulfate de magnésium, filtré et évaporé à sec. On recueille après chromatographie sur colonne de silice élué avec un mélange d'heptane et d'acétate d'éthyle (50:50), 650 mg (65%) du mélange (Z + E) des composés attendus.

### EXEMPLE 24

### Acides 4-{2-[3-adamantan-1-yl-4-(3-carbamoylmethoxy-propyl)-phenyl]-propenyl}-benzoïques.

De manière analogue à l'exemple 2 à partir de 650 mg (1,26 mmole) du mélange de composés (E) et (Z) obtenus à l'exemple 23, on recueille après trituration dans un mélange composé de 20% d'éther éthylique et 80% d'heptane, 340 mg (55%) du mélange (Z + E) des composés attendus.

### EXEMPLE 25

L' activité antagoniste des composés de formule(I) est évaluée dans le test de différenciation des cellules F9 de tératocarcinome embryonnaire de souris (Cancer Research 43, p 5268, 1983).

Ces composés testés à 10⁻⁶ M sont inactifs en tant qu'agonistes dans ce test et inhibent partiellement ou totalement l'effet produit par un rétinoide agoniste sur la morphologie et sur la sécrétion de l'activateur du plasminogène selon le protocole suivant.

Les cellules F9 sont ensemencées dans des clusters 12 puits, les composés sont testés de 10⁻⁹ à 10⁻⁵ M en présence de l'acide all-trans-retinoïque ou d'un rétinoide agoniste synthétique l'acide 4-(5,6,7,8-tétrahydro-5,5,8,8-tétraméthyl-2-naphtylaminométhyl)benzoïque (CD 2043) à 10⁻⁸ M. Après trois jours d'incubation, on effectue les observations morphologiques et l'on détermine la concentration du composé testé (IC50) qui inhibe de 50% l'effet de l'agoniste sur la sécrétion de l'activateur du plasminogène.

| Ex. No. | Antagoniste contre le CD 2043 (10nM) test de différenciation F9 IC50 (nM) |
|---|---|
| 2 | 310 |
| 4 | 9 |
| 5 | 630 |
| 10 | 35 |
| 7 | 600 |

### B. EXEMPLES DE FORMULATION

### 1) VOIE ORALE

(a) On prépare la composition suivante sous la forme d'un comprimé de 0,8 g

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administrera à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p. | 5 ml |

Pour le traitement de l'acné, on administrera à un individu adulte 1 ampoule par jour pendant 3 mois selon la gravité du cas traité.
(c) On prépare la formulation suivante destinée à être conditionnée en gélules :

| | |
|---|---|
| Composé de l'exemple 4 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administrera à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 1 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 jours.
(b) On prépare un gel en réalisant la formulation suivante :

| | |
|---|---|
| Composé de l'exemple 3 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel sera appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 2 | 0,030 g |
| Propylène glycol | 5,000 g |
| Butylhydroxytoluène | 0,100 g |
| Ethanol (à 95°) q.s.p | 100,000 g |

Cette lotion sera appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 12 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition sera appliquée quotidiennement, elle permet de lutter contre le vieillissement photoinduit.
(e) On prépare la crème Huile dans l'Eau non ionique suivante :

| | |
|---|---|
| Composé de l'exemple 14 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p | 100,000 g |

Cette crème sera appliquée sur une peau psoriatique 1 à 2 fois par jour pendant 30 Jours.
(f) On prépare un gel topique en procédant au mélange des ingrédients suivants :

| | |
|---|---|
| Composé de l'exemple 18 | 0,050 g |
| Ethanol 43,000 | g |
| a -tocophérol | 0,050 g |
| Polymère carboxyvinylique vendu sous la dénomination | |
| "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel sera appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 15 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On appliquera cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 5 | 0,050 g |
| Acide rétinoïque | 0,010 g |
| Mélange de stéarates de glycérol et de polyéthylène glycol (75 moles) vendu sous le nom de "Gelot 64" .. par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000g |

Cette crème sera appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 20 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société | |
| "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000g |

Cette crème sera appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 23 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" . par la société "DYNAMIT NOBEL" | 4,000 g |
| Eau q.s.p | 100,000 g |

Cette crème sera appliquée 1 fois par jour, elle aide à lutter contre le vieillissement qu'il soit photoinduit ou chronologique.

## Revendications

1. Composés stilbéniques, caractérisés par le fait qu'ils répondent à la formule générale (I) suivante : 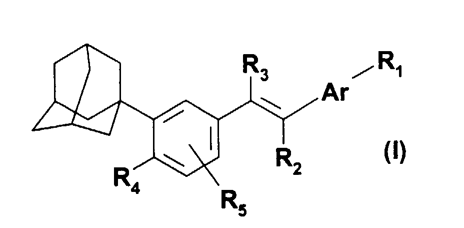 dans laquelle:
- R₁ représente
(i) le radical -CH₃,
(ii) le radical -CH₂-O-R₆,
(iii) le radical -O-R₆, ou
(iv) le radical -CO-R₇,
les radicaux R₆ et R₇ ayant les significations données ci-après,
- Ar représente un radical choisi parmi les radicaux de formules (a) à (f) suivantes: 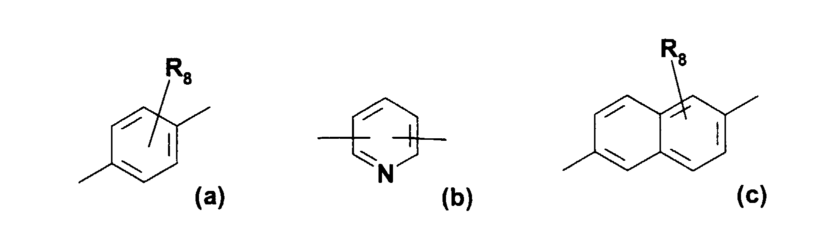 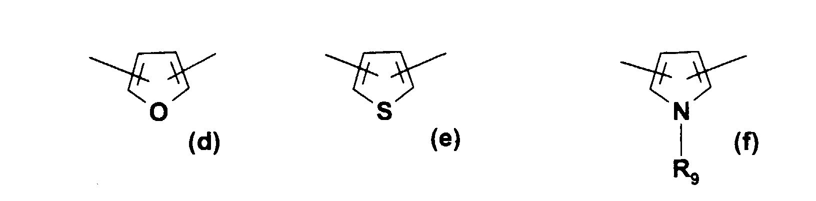 R₈ et R₉ ayant les significations données ci-après
- R₂ et R₃, identiques ou différents, représentent un atome d'hydrogène, un radical alkyle choisi parmi les radicaux méthyle, éthyle, propyle, isopropyle, tertiobutyle, ou hexyle,
- R₄ représente le radical -(X)m-(CH₂)ₙ-Y-(CH₂)ₚ-R₁₀
les valeurs m, n et p et les radicaux X, Y et R₁₀ ayant les significations données ci-après,
- R₅ représente un atome d'hydrogène, d'halogène, un radical alkyle choisi parmi les radicaux méthyle, éthyle, propyle, isopropyle, tertiobutyle, ou hexyle, ou un radical -O-R₆,
- R₆ représente un atome d'hydrogène, un radical alkyle choisi parmi les radicaux méthyle, éthyle, propyle, isopropyle, tertiobutyle, ou hexyle ou un radical -CO-R₁₁,
- R₇ représente un atome d'hydrogène, un radical alkyle choisi parmi les radicaux méthyle, éthyle, propyle, isopropyle, tertiobutyle, ou hexyle, un radical -OR₁₂ ou un radical 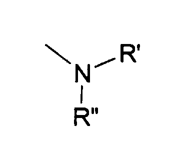 dans lequel R' et R", identiques ou différents, représentent un atome d'hydrogène ; un radical alkyle choisi parmi les radicaux méthyle, éthyle, propyle, isopropyle, tertiobutyle, ou hexyle; un radical mono ou polyhydroxyalkyle ; un radical phényle éventuellement substitué par au moins un atome d'halogène, un radical hydroxyle, un radical alkyle, une fonction nitro, un groupe méthoxy ou une fonction amine éventuellement substituée ; un reste de sucre choisi parmi un reste de glucose, de galactose, de mannose ou d'acide glucuronique, un reste d'aminoacide choisi parmi un reste dérivant de la lysine, de la glycine ou de l'acide aspartique, ou un reste de dipeptide ou de tripeptide ou encore pris ensemble, forment un hétérocycle choisi dans le groupe constitué par les radicaux pipéridino, morpholino, pyrrolidino ou pipérazino, éventuellement substitués en position 4 par un radical alkyle en C₁-C₆ ou polyhydroxyalkyle,
étant entendu que dans tout ce qui précède :
- m est un nombre entier égal à 0 ou 1,
- n est un nombre entier compris inclusivement entre 1 et 6,
- p est un nombre entier compris inclusivement entre 1 et 6,
- X représente O ou S(O)q,
- Y représente O, S(O)q ou N-R₉,
- q est un nombre entier compris inclusivement entre 0 et 2,
- R₈ représente un atome d'hydrogène, d'halogène, un radical alkyle choisi parmi les radicaux méthyle, éthyle, propyle, isopropyle, tertiobutyle, ou hexyle, ou un radical -O-R₆,
- R₉ représente un atome d'hydrogène, un radical alkyle choisi parmi les radicaux méthyle, éthyle, propyle, isopropyle, tertiobutyle, ou hexyle, ou un radical -CO-R₁₁,
- R₁₀ représente un radical mono ou polyhydroxyalkyle dont les hydroxy sont éventuellement protégés sous forme de méthoxy, éthoxy ou d'acétoxy ou d'acétonide, un radical -CO-R₇, un radical phényle éventuellement substitué par au moins un atome d'halogène, un radical hydroxyle, un radical alkyle, une fonction nitro, un groupe méthoxy ou une fonction amine éventuellement substituée ou aralkyle éventuellement substitué,
- R₁₁ représente un radical alkyle choisi parmi les radicaux méthyle, éthyle, propyle, isopropyle, tertiobutyle, ou hexyle,
- R₁₂ représente un atome d'hydrogène, un radical alkyle, un radical alkényle, un radical mono ou polyhydroxyalkyle dont les hydroxy sont éventuellement protégés sous forme de méthoxy, éthoxy ou d'acétoxy ou d'acétonide, un radical phényle éventuellement substitué par au moins un atome d'halogène, un radical hydroxyle, un radical alkyle, une fonction nitro, un groupe méthoxy ou une fonction amine éventuellement substituée ou aralkyle éventuellement substitué, ou un reste de sucre choisi parmi un reste de glucose, de galactose, de mannose ou d'acide glucuronique, un reste d'aminoacide choisi parmi un reste dérivant de la lysine, de la glycine ou de l'acide aspartique, ou un reste de dipeptide ou de tripeptide,
ainsi que leurs sels et leurs isomères optiques et géométriques.

2. Composés selon la revendication 1, caractérisés par le fait qu'ils se présentent sous forme de sels d'un métal alcalin ou alcalino-terreux, de zinc, d'une amine organique, d'un acide minéral ou organique.

3. Composés selon l'une des revendications précédentes, caractérisés en ce que les radicaux polyhydroxyalkyles sont choisis parmi le groupe constitué par les radicaux 2,3-dihydroxypropyle, 2,3,4-trihydroxybutyle, 2,3,4,5-tétrahydroxypentyle ou le reste du pentaérythritol.

4. Composés selon l'une des revendications précédentes, caractérisés en ce que les radicaux aralkyles sont choisis parmi le groupe constitué par les radicaux benzyle ou phénéthyle, éventuellement substitués par au moins un atome d'halogène, un hydroxyle, une fonction nitro ou un groupe méthoxy.

5. Composés selon l'une des revendications précédentes, caractérisés en ce que les radicaux alkényles sont choisis dans le groupe constitué par les radicaux contenant de 2 à 5 atomes de carbone et présentant une ou plusieurs insaturations éthyléniques, et en particulier le radical allyle.

6. Composés selon l'une quelconque des revendications précédentes, caractérisés en ce que les atomes d'halogène sont choisis dans le groupe constitué par le fluor, le brome et le chlore.

7. Composés selon la revendication 1, caractérisés par le fait qu'ils sont pris, seuls ou en mélanges, dans le groupe constitué par :
4-[(E)-2-(3-(1-adamantyl)-4-méthoxyéthoxyméthoxyphényl)éthényl]benzoate d'éthyle ;
Acide 4-[(E)-2-(3-(1-adamantyl)-4-methoxyethoxymethoxyphényl) éthényl]benzoïque ;
4-[(E)-2-(3-(1-adamantyl)-4-méthoxyéthoxyméthoxyphényl)-1-propényl] benzoate d'éthyle .
Acide 4-[(E)-2-(3-(1-adamantyl)-4-methoxyethoxymethoxyphényl)-1-propényl] benzoïque ;
Acide 4-[(Z)-2-(3-(1-adamantyl)-4-methoxyethoxymethoxyphényl)-1-propényl] benzoïque ;
5-{2-[3-Adamantan-1-yl-4-(2-méthoxy-éthoxyméthoxy)-phényl]-propèn-(E)-yl}-pyridine-2-carboxylate de méthyle ;
Acide 5-{2-[3-adamantan-1-yl-4-(2-méthoxy-éthoxyméthoxy)-phényl]-propèn-(E)-yl}-pyridine-2-carboxylique ;
Acide 5-{2-[3-adamantan-1-yl-4-(2-méthoxy-éthoxyméthoxy)-phényl]-propèn-(Z)-yl}-pyridine-2-carboxylique ;
6-{2-[3-Adamantan-1-yl-4-(2-méthoxy-éthoxyméthoxy)-phényl]-propèn-(E)-yl}-nicotinate d'éthyle ;
Acide 6-{2-[3-adamantan-1-yl-4-(2-méthoxy-éthoxyméthoxy)-phényl]-propèn-(E)-yl}-nicotinique ;
Acide 6-{2-[3-Adamantan-1-yl-4-(2-méthoxy-éthoxyméthoxy)-phényl]-propèn-(Z)-yl}-nicotinique ;
4-{2-[3-Adamantan-1-yl-4-(2-méthoxy-éthoxyméthoxy)-phényl]-propèn-(Z)-yl}-2-méthoxybenzoate de méthyle ;
Acide 4-{2-[3-adamantan-1-yl-4-(2-méthoxy-éthoxyméthoxy)-phényl]-propèn-(E)-yl}-2-méthoxy-benzoique ;
Acide 4-{2-[3-adamantan-1-yl-4-(2-méthoxy-éthoxyméthoxy)-phényl]-propèn-(Z)-yl}-2-méthoxy-benzoique ;
4-{2-[3-adamantan-1-yl-4-(3-éthoxyméthoxy-propyl)-phényl]-propèn-(E/Z)-yl}-benzoate d'éthyle ;
Acide 4-{2-[3-adamantan-1-yl-4-(3-éthoxyméthoxy-propyl)-phényl]-propèn-(E)-yl}-benzoïque;
Acide 4-{2-[3-adamantan-1-yl-4-(3-éthoxyméthoxy-propyl)-phényl]-propèn-(Z)-yl}-benzoïque ;
4-{2-[3-adamantan-1-yl-4-(3-benzyloxy-propyl)-phényl]-propèn-(E/Z)-yl}-benzoate d'éthyle ;
Acide 4-{2-[3-adamantan-1-yl-4-(3-benzyloxy-propyl)-phényl]-propèn-(E)-yl}-benzoïque ;
Acide 4-{2-[3-adamantan-1-yl-4-(3-benzyloxy-propyl)-phényl]-propèn-(Z)-yl}-benzoïque ;
4-{2-[3-Adamantan-1-yl-4-(3-diethylcarbamoylmethoxy-propyl)-phenyl]-propenyl}-benzoate d'éthyle ;
Acide 4-{2-[3-Adamantan-1-yl-4-(3-diethylcarbamoylmethoxy-propyl)-phenyl]-propenyl}-benzoïque ;
4-{2-[3-adamantan-1-yl-4-(3-carboxymethoxy-propyl)-phenyl]-propenyl}-benzoate d'éthyle ;
Acide 4-{2-[3-adamantan-1-yl-4-(3-carboxymethoxy-propyl)-phenyl]-propenyl}-benzoïque ;
4-{2-[3-adamantan-1-yl-4-(3-carbamoylmethoxy-propyl)-phenyl]-propenyl}-benzoate d'éthyle ;
Acide 4-{2-[3-adamantan-1-yl-4-(3-carbamoylmethoxy-propyl)phenyl] propenyl} benzoïque ;
N-éthyl- 4-[(E)-2-(3-(1-adamantyl)-4-methoxyethoxymethoxyphényl)-1-propényl] benzamide ;
4-[(E)-2-(3-(1-adamantyl)-4-methoxyethoxymethoxyphényl)-1-propényl] benzamide ;
N-4-(hydroxyphenyl)-4-[(E)-2-(3-(1-adamantyl)-4-methoxyethoxymethoxyphényl)-1-propényl] benzamide ;
4-[(E)-2-(3-(1-adamantyl)-4-methoxyethoxymethoxyphényl)-1-propényl] benzèneméthanol ;
4-[(E)-2-(3-(1-adamantyl)-4-methoxyethoxymethoxyphényl)-1-propényl] benzaldéhyde ;
4-[(E)-2-(3-(1-adamantyl)-4-methoxyethoxymethoxyphényl)-1-propényl]phénol ;
Morpholide de l'acide 4-[(E)-2-(3-(1-adamantyl)-4-methoxyethoxymethoxyphényl)-1-propényl] benzoïque ; et
Acide 4-[(E)-2-(3-(1-adamantyl)-4-methoxyethoxymethylsulfanylphényl)-1-propényl] benzoïque.

8. Composés selon la revendication 1, caractérisés par le fait qu'ils présentent l'une au moins des caractéristiques suivantes :
- R₁ est le radical -CO-R₇
- Ar représente les radicaux de formule (a) ou (b),
- X et Y, identiques ou différents, représentent des atomes d'oxygène ou de soufre,
- R₃ représente un radical alkyle choisi parmi les radicaux méthyle, éthyle, propyle, isopropyle, tertiobutyle, ou hexyle.

9. Composés selon l'une quelconque des revendications précédentes pour une utilisation comme médicament.

10. Utilisation d'un composé selon l'une quelconque des revendications 1 à 8 pour la fabrication d'un médicament destiné au traitement d'une pathologie choisie parmi :
- les acnés vulgaires, comédoniennes, polymorphes, rosacées, les acnés nodulokystiques, conglobata, les acnés séniles, les acnés secondaires, l'acné solaire, médicamenteuse ou professionnelle,
- les ichtyoses, les états ichtyosiformes, la maladie de Darrier, les kératodermies palmoplantaires, les leucoplasies et les états leucoplasiformes, le lichen cutané ou muqueux (buccal),
- le psoriasis cutané, muqueux, unguéal, le rhumatisme psoriatique ; l'atopie cutanée, l'eczéma l'atopie respiratoire ou l'hypertrophie gingivale ;
- les proliférations dermiques ou épidermiques bénignes ou malignes, d'origine virale ou non comprenant les verrues vulgaires, les verrues planes et l'épidermodysplasie verruciforme, les papillomatoses orales, les florides, ou les proliférations pouvant être induites par les ultra-violets comprenant les épithélioma baso et spinocellulaires,
- les dermatoses bulleuses et les maladies du collagène,
- les cornéopathies,
- le vieillissement de la peau, qu'il soit photo-induit ou chronologique,
- les pigmentations et les kératoses actiniques, ou toutes pathologies associées au vieillissement chronologique ou actinique,
- l'atrophie cutanée,
- les stigmates de l'atrophie épidermique et/ou dermique induite par les corticostéroïdes locaux ou systémiques,
- les troubles de la cicatrisation ou les vergetures,
- les troubles de la fonction sébacée comprenant l'hyperséborrhée de l'acné ou la séborrhée simple,
- les états cancéreux ou précancéreux,
- les leucémies promyélocytaires,
- l'arthrite,
- le syndrome de Kaposis,
- l'alopécie,
- l'artériosclérose ou l'hypertension ainsi que le diabète non-insulino dépendant,
- les désordres cutanés dus à une exposition aux rayonnements U.V.

11. Utilisation d'un composé selon l'une quelconque des revendications 1 à 8 pour la fabrication d'un médicament destiné à la prévention d'une pathologie choisie parmi :
- l'atopie cutanée, l'eczéma, l'atopie respiratoire ou l'hypertrophie gingivale ;
- le vieillissement de la peau, qu'il soit photo-induit ou chronologique,
- l'atrophie cutanée,
- les stigmates de l'atrophie épidermique et/ou dermique induite par les corticostéroïdes locaux ou systémiques,
- les troubles de la cicatrisation
- les vergetures,
- les états cancéreux ou précancéreux,
- les leucémies promyélocytaires,
- l'alopécie,
- l'artériosclérose ou l'hypertension ainsi que le diabète non-insulino dépendant,
- les désordres cutanés dus à une exposition aux rayonnements U.V.

12. Composition pharmaceutique, caractérisée par le fait qu'elle comprend, dans un support pharmaceutiquement acceptable, au moins l'un des composés tels que définis à l'une quelconque des revendications 1 à 8.

13. Composition selon la revendication 12 caractérisée en ce que la concentration en composé(s) selon l'une des revendication 1 à 8 est comprise entre 0,001 % et 5 % en poids par rapport à l'ensemble de la composition.

14. Composition cosmétique, caractérisée par le fait qu'elle comprend, dans un support cosmétiquement acceptable, au moins l'un des composés tels que définis à l'une quelconque des revendications 1 à 8.

15. Composition selon la revendication 14 caractérisée en ce que la concentration en composé(s) selon l'une des revendications 1 à 8 est comprise entre 0,001 % et 3 % en poids par rapport à l'ensemble de la composition.

16. Utilisation d'une composition cosmétique telle que définie à l'une des revendications 14 ou 15 pour l'hygiène corporelle ou capillaire.

## Claims

1. Stilbene compounds, characterized in that they correspond to the general formula (I) below: 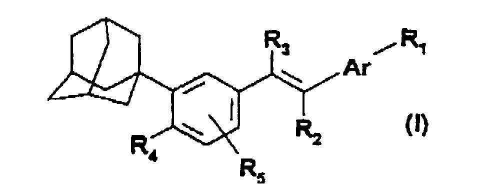 in which:
- R₁ represents
(i) the -CH₃ radical,
(ii) the radical -CH₂-O-R₆,
(iii) the radical -O-R₆, or
(iv) the radical -CO-R₇,
the radicals R₆ and R₇ having the meanings given below,
- Ar represents a radical chosen from the radicals of formulae (a) to (f) below: 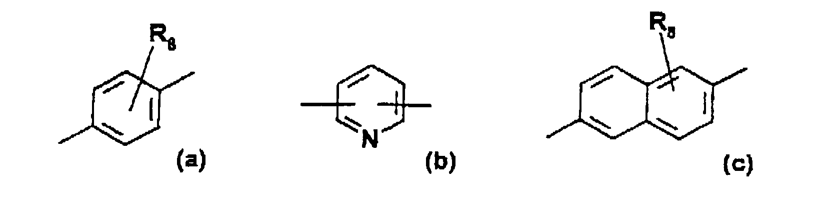 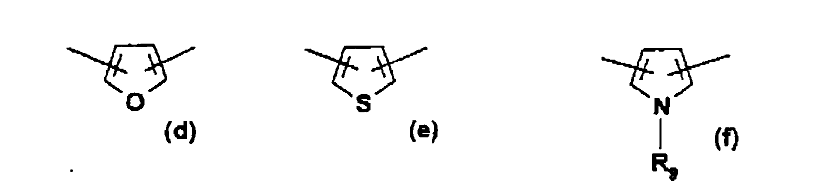 R₈ and R₉ having the meanings given below
- R₂ and R₃, which may be identical or different, represent a hydrogen atom or an alkyl radical chosen from methyl, ethyl, propyl, isopropyl, tert-butyl and hexyl radicals,
- R₄ represents the radical- (X)m-(CH₂)n-Y-(CH₂)p-R₁₀
the values m, n and p and the radicals X, Y and R₁₀ having the meanings given below,
- R₅ represents a hydrogen or halogen atom, an alkyl radical chosen from methyl, ethyl, propyl, isopropyl, tert-butyl and hexyl radicals, or a radical -O-R₆,
- R₆ represents a hydrogen atom, an alkyl radical chosen from methyl, ethyl, propyl, isopropyl, tert-butyl or hexyl radicals, or a radical -CO-R₁₁,
- R₇ represents a hydrogen atom, an alkyl radical chosen from methyl, ethyl, propyl, isopropyl, tert-butyl and hexyl radicals, a radical -OR₁₂ or a radical 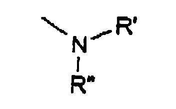 in which R' and R", which may be identical or different, represent a hydrogen atom, an alkyl radical chosen from methyl, ethyl, propyl, isopropyl, tert-butyl and hexyl radicals; a mono- or polyhydroxyalkyl radical; a phenyl radical optionally substituted with at least one halogen atom, a hydroxyl radical, an alkyl radical, a nitro function, a methoxy group or an optionally substituted amine function; a sugar residue chosen from a glucose, galactose, mannose and glucuronic acid residue, an amino acid residue chosen from a residue derived from lysine, glycine or aspartic acid, or a dipeptide or tripeptide residue or alternatively, taken together, form a heterocycle chosen from the group consisting of piperidino, morpholino, pyrrolidino and piperazino radicals, optionally substituted in position 4 with a C₁-C₆ alkyl or polyhydroxyalkyl radical,
it being understood that, in all of the text hereinabove:
- m is an integer equal to 0 or 1,
- n is an integer between 1 and 6 inclusive,
- p is an integer between 1 and 6 inclusive,
- X represents O or S(O)q,
- Y represents 0, S(O)q or N-R₉,
- q is an integer between 0 and 2 inclusive,
- R₈ represents a hydrogen or halogen atom, an alkyl radical chosen from methyl, ethyl, propyl, isopropyl, tert-butyl and hexyl radicals, or a radical -O-R₆,
- R₉ represents a hydrogen atom, an alkyl radical chosen from methyl, ethyl, propyl, isopropyl, tert-butyl and hexyl radicals, or a radical -CO-R₁₁,
- R₁₀ represents a mono- or polyhydroxyalkyl radical in which the hydroxyls are optionally protected in the form of methoxy, ethoxy, acetoxy or acetonide, a radical -CO-R₇, a phenyl radical optionally substituted with at least one halogen atom, a hydroxyl radical, an alkyl radical, a nitro function, a methoxy group or an optionally substituted amine or optionally substituted aralkyl function,
- R₁₁ represents an alkyl radical chosen from methyl, ethyl, propyl, isopropyl, tert-butyl and hexyl radicals,
- R₁₂ represents a hydrogen atom, an alkyl radical, an alkenyl radical, a mono- or polyhydroxyalkyl radical in which the hydroxyls are optionally protected in the form of methoxy, ethoxy, acetoxy or acetonide, a phenyl radical optionally substituted with at least one halogen atom, a hydroxyl radical, an alkyl radical, a nitro function, a methoxy group or an optionally substituted amine or optionally substituted aralkyl function, or a sugar residue chosen from a glucose, galactose, mannose and glucuronic acid residue, an amino acid residue chosen from a residue derived from lysine, glycine or aspartic acid, or a dipeptide or tripeptide residue, as well as the salts thereof and the optical and geometrical isomers thereof.

2. Compounds according to Claim 1, characterized in that they are in the form of salts of an alkali metal or alkaline-earth metal, of zinc, of an organic amine or of an inorganic or organic acid.

3. Compounds according to one of the preceding claims, characterized in that the polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

4. Compounds according to one of the preceding claims, characterized in that the aralkyl radicals are chosen from the group consisting of benzyl or phenethyl radicals, optionally substituted with at least one halogen atom, a hydroxyl, a nitro function or a methoxy group.

5. Compounds according to one of the preceding claims, characterized in that the alkenyl radicals are chosen from the group consisting of radicals containing from 2 to 5 carbon atoms and having one or more ethylenic unsaturations, and in particular the allyl radical.

6. Compounds according to any one of the preceding claims, characterized in that the halogen atoms are chosen from the group consisting of fluorine, bromine and chlorine.

7. Compounds according to Claim 1, characterized in that they are taken, alone or as mixtures, from the group consisting of:
Ethyl 4-[(E)-2-(3-(1-adamantyl)-4-methoxyethoxymethoxyphenyl)ethenyl]benzoate.
4-[(E)-2-(3-(1-Adamantyl)-4-methoxyethoxymethoxyphenyl)ethenyl]benzoic acid.
Ethyl 4-(E)-2-(3-(1-adamantyl)-4-methoxyethoxymethoxyphenyl)-1-propenyl]benzoate.
4-[(E)-2-(3-(1-Adamantyl)-4-methoxyethoxymethoxyphenyl)-1-propenyl]benzoic acid.
4-[(Z)-2-(3-(1-Adamantyl)-4-methoxyethoxymethoxyphenyl)-1-propenyl]benzoic acid.
Methyl 5-{2-[3-adamant-1-yl-4-(2-methoxyethoxymethoxy)phenyl]propen-(E)-yl}pyridine-2-carboxylate.
5-{2-[3-Adamant-1-yl-4-(2-methoxyethoxymethoxy)-phenyl]propen-(E)-yl}pyridine-2-carboxylic acid.
5-{2-[3-Adamant-1-yl-4-(2-methoxyethoxymethoxy)-phenyl]propen-(Z)-yl)pyridine-2-carboxylic acid.
Ethyl 6-{2-[3-adamant-1-yl-4-(2-methoxyethoxymethoxy)phenyl]propen-(E)-yl}nicotinate.
6-{2-[3-Adamant-1-yl-4-(2-methoxyethoxymethoxy)-phenyl]propen-(E)-yl)nicotinic acid.
6-{2-[3-Adamant-1-yl-4-(2-methoxyethoxymethoxy)-phenyl]propen-(Z)-yl}nicotinic acid.
Methyl 4-{2-[3-adamant-1-yl-4-(2-methoxyethoxymethoxy)phenyl]propen-(Z)-yl}-2-methoxybenzoate.
4-{2-[3-Adamant-1-yl-4-(2-methoxyethoxymethoxy)-phenyl]propen-(E)-yl}-2-methoxybenzoic acid.
4-{2-[3-Adamant-1-yl-4-(2-methoxyethoxymethoxy)phenyl]propen- (Z)-yl}-2-methoxybenzoic acid.
Ethyl 4-{2-[3-adamant-1-yl-4-(3-ethoxymethoxypropyl)phenyl]propen-(E/z)-yl}benzoate.
4-{2-[3-Adamant-1-yl-4-(3-ethoxymethoxypropyl)-phenyl]propen-(E)-yl}benzoic acid.
4-{2-[3-Adamant-1-yl-4-(3-ethoxymethoxypropyl)-phenyl]propen-(Z)-yl}benzoic acid.
Ethyl 4-{2-[3-adamant-1-yl-4-(3-benzyloxypropyl)-phenyl]propen- (E/Z)-yl}-benzoate.
4-{2-[3-Adamant-1-yl-4-(3-benzyloxypropyl)phenyl]-propen-(E)-yl}-benzoic acid.
4-{2-[3-Adamant-1-yl-4- (3-benzyloxypropyl)phenyl]-propen-(Z)-yl}benzoic acid.
Ethyl 4-(2-[3-adamant-1-yl-4-(3-diethylcarbamoylmethoxypropyl)phenyl]propenyl)benzoate.
4-{2-[3-Adamant-l-yl-4-(3-diethylcarbamoylmethoxypropyl)phenyl]propenyl)benzoic acid.
Ethyl 4-{2-[3-adamant-1-yl-4-(3-carboxymethoxypropyl)phenyl]propenyl]benzoate.
4-{2-[3-Adamant-1-yl-4-(3-carboxymethoxypropyl)-phenyl]propenyl)benzoic acid.
Ethyl 4-{2-[3-adamant-1-yl-4-(3-carbamoylmethoxypropyl)phenyl]propenyl}benzoate.
4-{2-[3-Adamant-1-yl-4-(3-carbamoylmethoxypropyl)-phenyl]propenyl)benzoic acid.
N-Ethyl-4-(E)-2-(3-(1-adamantyl)-4-methoxyethoxymethoxyphenyl)-1-propenyl]benzamide.
4-[(E)-2-(3-(1-Adamantyl)-4-methoxyethoxymethoxyphenyl)-1-propenyl]benzamide.
N-4- (Hydroxyphenyl)-4-[(E) -2-(3-(1-adamantyl)-4-methoxyethoxymethoxyphenyl)-1-propenyl] benzamide.
4-[(E)-2-(3-(1-Adamantyl)-4-methoxyethoxymethoxyphenyl)-1-propenyl)benzenemethanol,
4-[(E)-2-(3-(1-Adamantyl)-4-methoxyethoxymethoxyphenyl)-1-propenyl]benzaldehyde.
4-[(E)-2-(3-(1-Adamantyl)-4-methoxyethoxymethoxyphenyl)-1-propenyl]phenol.
4-[(E)-2-(3-(1-Adamantyl)-4-methoxyethoxymethoxyphenyl)-1-propenyl]benzoic acid morpholide, and
4-(E)-2-(3-(1-Adamantyl)-4-methoxyethoxymethylsulphanylphenyl)-1-propenyl]benzoic acid.

8. Compounds according to Claim 1, characterized in that they have at least one of the following characteristics:
- R₁ is the radical -CO-R₇,
- Ar represents the radicals of formula (a) or (b),
- X and Y, which may be identical or different, represent oxygen or sulphur atoms,
- R₃ represents an alkyl radical chosen from methyl, ethyl, propyl, isopropyl, tert-butyl and hexyl radicals.

9. Compounds according to any one of the preceding claims, for use as medicinal products.

10. Use of a compound according to any one of Claims 1 to 8, for the manufacture of a medicinal product intended for treating a pathology chosen from:
- common acne, comedones, polymorphonuclear leukocytes, acne rosacea, nodulocystic acne, acne conglobata, senile acne, secondary acnes and solar acne, medication-induced acne or occupational acne,
- ichthyosis, ichthyosiform states, Darrier's disease, palmoplantar keratoderma, leucoplasias and leucoplasiform states, and cutaneous or mucous (buccal) lichen,
- cutaneous, mucous or ungual psoriasis, psoriatic rheumatism; cutaneous atopy, eczema, respiratory atopy or gingival hypertrophy;
- benign or malignant dermal or epidermal proliferations, of viral origin or otherwise, including common warts, flat warts and verruciform epidermodysplasia, oral or florid papillomatoses, or proliferations which can be induced by ultraviolet radiation, including basocellular and spinocellular epitheliomas,
- bullosis and collagen diseases,
- corneopathies,
- both light-induced and chronological ageing of the skin,
- pigmentations and actinic keratoses, or any pathology associated with chronological or actinic ageing,
- skin atrophy,
- the stigmata of epidermal and/or dermal atrophy induced by local or systemic corticosteroids,
- cicatrization disorders or stretchmarks,
- disorders of sebaceous functioning including the hyperseborrhoea of acne or simple seborrhoea,
- cancerous or precancerous conditions,
- promyelocytic leukaemias,
- arthritis,
- Kaposi's syndrome,
- alopecia,
- arteriosclerosis or hypertension and non-insulin-dependent diabetes,
- skin disorders due to exposure to UV radiation.

11. Use of a compound according to any one of Claims 1 to 8, for the manufacture of a medicinal product intended for preventing a pathology chosen from:
- cutaneous atopy, eczema, respiratory atopy or gingival hypertrophy;
- light-induced and chronological ageing of the skin;
- skin atrophy;
- the stigmata of epidermal and/or dermal atrophy induced by local or systemic corticosteroids;
- cicatrization disorders;
- stretchmarks;
- cancerous or precancerous conditions;
- promyelocytic leukaemias;
- alopecia;
- arteriosclerosis or hypertension and non-insulin-dependent diabetes;
- skin disorders due to exposure to UV radiation.

12. Pharmaceutical composition, characterized in that it comprises, in a pharmaceutically acceptable support, at least one of the compounds as defined in any one of Claims 1 to 8.

13. Composition according to Claim 12, characterized in that the concentration of compound(s) according to one of Claims 1 to 8 is between 0.001% and 5% by weight relative to the composition as a whole.

14. Cosmetic composition, characterized in that it comprises, in a cosmetically acceptable support, at least one of the compounds as defined in any one of Claims 1 to 8.

15. Composition according to Claim 14, characterized in that the concentration of compound(s) according to one of Claims 1 to 8 is between 0.001% and 3% by weight relative to the composition as a whole.

16. Use of a cosmetic composition as defined in either of Claims 14 or 15, for body or hair hygiene.

## Patentansprüche

1. Stilbenverbindungen, dadurch gekennzeichnet, daß sie der allgemeinen Formel (I) entsprechen: 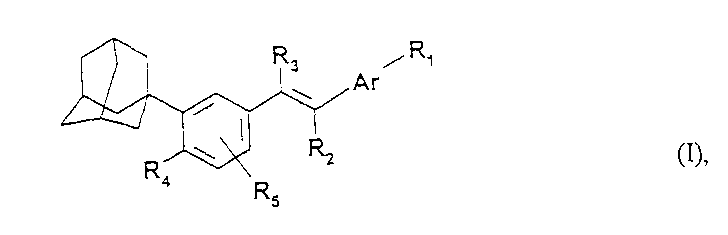 worin bedeuten:
- R₁
(i) die Gruppe -CH₃,
(ii) eine Gruppe -CH₂-O-R₆,
(iii) eine Gruppe -O-R₆,
(iii) eine Gruppe -CO-R₇,
wobei R₆ und R₇ die nachfolgend angegebenen Bedeutungen aufweisen;
- Ar eine Gruppe, die unter den Gruppen der folgenden Formeln (a) bis (f) ausgewählt ist. 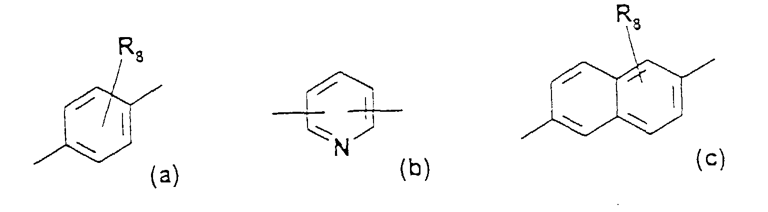 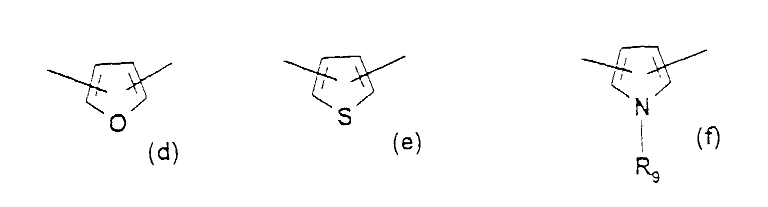 wobei R₈ und R₉ die nachstehend angegebenen Bedeutungen aufweisen;
- R₂ und R₃, die identisch oder voneinander verschieden sind, ein Wasserstoffatom oder eine Alkylgruppe, die unter Methyl, Ethyl, Propyl, Isopropyl, *tert.*-Butyl oder Hexyl ausgewählt ist;
- R₄ die Gruppe -(X)ₘ-(CH₂)ₙ-Y-(CH₂)ₚ-R₁₀, wobei m, n, p, X, Y und R₁₀ nachstehend definiert sind;
- R₅ Wasserstoff, Halogen, eine Alkylgruppe, die unter Methyl, Ethyl, Propyl, Isopropyl, tert.-Butyl oder Hexyl ausgewählt ist, oder -O-R₆;
- R₆ Wasserstoff, eine Alkylgruppe, die unter Methyl, Ethyl, Propyl, Isopropyl, *tert.*-Butyl oder Hexyl ausgewählt ist, oder -CO-R₁₁;
- R₇ Wasserstoff, eine Alkylgruppe, die unter Methyl, Ethyl, Propyl, Isopropyl, *tert.*-Butyl oder Hexvl ausgewählt ist, -O-R₁₂ oder eine Gruppe 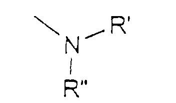 worin R' und R", die identisch oder voneinander verschieden sind, Wasserstoff, eine Alkylgruppe, die unter Methyl, Ethyl, Propyl, Isopropyl, *tert.*-Butyl oder Hexyl ausgewählt ist, eine Mono- oder Polyhydroxyalkylgruppe, eine Phenylgruppe, die gegebenenfalls mit mindestens einem Halogenatom, einer Hydroxygruppe, einer Alkylgruppe, einer Nitrogruppe, einer Methoxygruppe oder einer gegebenenfalls substituierten Aminogruppe substituiert ist, einen Zuckerrest, der unter den Resten von Glucose, Galactose, Mannose oder Glucuronsäure ausgewählt ist, einen Aminosäurerest, der unter einem von Lysin, Glycin oder Asparaginsäure abgeleiteten Rest ausgewählt ist, oder einen Di- oder Tripeptidrest; oder R' und R" bilden gemeinsam einen Heterocyclus, der unter Piperidino, Morpholino, Pyrrolidino oder Piperazino ausgewählt ist, die gegebenenfalls in 4-Stellung mit einer C₁₋₆-Alkylgruppe oder Polyhydroxyalkylgruppe substituiert sind;
mit der Maßgabe, daß
- m 0 oder 1 ist,
- n eine ganze Zahl im Bereich von 1 bis 6 ist,
- p eine ganze Zahl im Bereich von 1 bis 6 ist,
- X O oder die Gruppe S(O)_{q} bedeutet,
- Y O, S(O)_{q} oder N-R₉ bedeutet,
- q 0, 1 oder 2 ist,
- R₈ Wasserstoff, Halogen, eine Alkylgruppe, die unter Methyl, Ethyl, Propyl, Isopropyl, *tert.* -Butyl oder Hexyl ausgewählt ist, oder eine Gruppe -O-R₆ bedeutet,
- R₉ Wasserstoff, eine Alkylgruppe, die unter Methyl, Ethyl, Propyl, Isopropyl, *tert.-Butyl* oder Hexyl ausgewählt ist, oder eine Gruppe -CO-R₁₁ bedeutet,
- R₁₀ eine Mono- oder Polyhydroxyalkylgruppe, wobei die Hydroxygruppen gegebenenfalls in Form von Methoxy, Ethoxy, Acetoxy oder Acetonid geschützt sind, eine Gruppe -CO-R₇, eine Phenylgruppe, die gegebenenfalls mit mindestens einem Halogenatom, einer Hydroxygruppe, einer Alkylgruppe, einer Nitrogruppe, einer Methoxygruppe oder einer gegebenenfalls substituierten Aminogruppe substituiert ist, oder eine gegebenenfalls substituierte Aralkylgruppe,
- R₁₁ eine Alkylgruppe, die unter Methyl, Ethyl, Propyl, Isopropyl, tert.-Butyl oder Hexyl ausgewählt ist,
- R₁₂ Wasserstoff, eine Alkylgruppe, eine Alkenylgruppe, eine Monohydroxyalkylgruppe oder eine Polyhydroxyalkylgruppe, deren Hydroxygruppen gegebenenfalls in Form von Methoxy, Ethoxy, Acetoxy oder Acetonid geschützt sind, eine Phenylgruppe, die gegebenenfalls mit mindestens einem Halogenatom, einer Hydroxygruppe, einer Alkylgruppe, einer Nitrogruppe, einer Methoxygruppe oder einer gegebenenfalls substituierten Aminogruppe substituiert ist, eine gegebenenfalls substituierte Aralkylgruppe, einen Zuckerrest, der unter Glucose, Galactose, Mannose oder Glucuronsäure ausgewählt ist, einen Aminosäurerest, der unter einem von Lysin, Glycin oder Asparaginsäure abgeleiteten Rest ausgewählt ist, oder einen Di- oder Tripeptidrest;
sowie die optischen oder geometrischen Isomere dieser Verbindungen sowie ihre Salze.

2. Verbindungen nach Anspruch 1, dadurch gekennzeichnet, daß sie in Form von Alkali-, Erdalkali- oder Zinksalzen, Salzen eines organischen Amins oder Salzen einer organischen oder anorganischen Säure vorliegen.

3. Verbindungen nach einem der vorhergehenden Ansprüche, dadurch gekennzeichnet, daß die Polyhydroxyalkylgruppen unter 2,3-Dihydroxy-propyl, 2,3,4-Trihydroxybutyl, 2,3,4,5-Tetrahydroxypentyl oder Pentaerythrit ausgewählt isnd.

4. Verbindungen nach einem der vorhergehenden Ansprüche, dadurch gekennzeichnet, daß die Aralkylgruppen unter Benzyl oder Phenethyl ausgewählt sind, die gegebenenfalls mit mindestens einem Halogenatom, einer Hydroxygruppe, einer Nitrogruppe oder einer Methoxygruppe substituiert sind.

5. Verbindungen nach einem der vorhergehenden Ansprüche, dadurch gekennzeichnet, daß die Alkenylgruppen unter den Gruppen mit 2 bis 5 Kohlenstoffatomen ausgewählt sind, die eine oder mehrer ethylenische Doppelbindungen aufweisen, und insbesondere Allyl.

6. Verbindungen nach einem der vorhergehenden Ansprüche, dadurch gekennzeichnet, daß die Halogenatome unter Fluor, Brom und Chlor ausgewählt sind.

7. Verbindungen nach einem der vorhergehenden Ansprüche, dadurch gekennzeichnet, daß sie unter den folgenden Verbindungen oder deren Gemischen ausgewählt sind:
- Ethyl-4-[(E)-2-(3-(1-adamantyl)-4-methoxyethoxymethoxyphenyl)ethenyl]-benzoat;
- 4-[(E)-2-(3-(1-Adamantyl)-4-methoxyethoxymethoxyphenyl)ethenyl]-benzoesäure;
- Ethyl-4-[(E)-2-(3-(1-adamantyl)-4-methoxyethoxymethoxyphenyl)-1-propenyl]-benzoat;
- 4-[(E)-2-(3-(1-Adamantyl)-4-methoxyethoxymethoxyphenyl)-1-propenyl]-benzoesäure;
- 4-[(Z)-2-(3-(1-Adamantyl)-4-methoxyethoxymethoxyphenyl)-1-propenyl]-benzoesäure;
- Methyl-5-{2-[3-adamantan-1-yl-4-(2-methoxyethoxymethoxy)-phenyl]-propen-(E)-yl}-pyridin-2-carboxylat;
- 5-{2-[3-Adamantan-1-yl-4-(2-methoxyethoxymethoxy)-phenyl]-propen-(E)-yl}-pyridin-2-carbonsäure;
- 5-{2-[3-Adamantan-1-yl-4-(2-methoxyethoxymethoxy)-phenyl]-propen-(Z)-yl}-pyridin-2-carbonsäure;
- Ethyl-6-{2-[3-adamantan-1-yl-4-(2-methoxyethoxymethoxy)-phenyl]-propen-(E)-yl}-nicotinat;
- 6-{2-[3-Adamantan-1-yl-4-(2-methoxyethoxymethoxy)-phenyl]-propen-(E)-yl}-nicotinsäure;
- 6-{2-[3-Adamantan-1-yl-4-(2-methoxyethoxymethoxy)-phenyl]-propen-(Z)-yl}-nicotinsäure;
- Methyl-4-{2-[3-adamantan-1-yl-4-(2-methoxyethoxymethoxy)-phenyl]-propen-(Z)-yl }-2-methoxy-benzoat;
- 4-{2-[3-Adamantan-1-yl-4-(2-methoxyethoxymethoxy)-phenyl]-propen-(E)-yl}-2-methoxy-benzoesäure;
- 4-{2-[3-Adamantan-1-yl-4-(2-methoxyethoxymethoxy)-phenyl]-propen-(Z)-yl}-2-methoxy-benzoesäure;
- Ethyl-4-{2-[3-adamantan-1-yl-4-(3-ethoxymethoxypropyl)-phenyl]-propen-(E/Z)-yl}-benzoat;
- 4-{2-[3-Adamantan-1-yl-4-(3-ethoxymethoxypropyl)-phenyl]-propen-(E)-yl}-benzoesäure;
- 4-{2-[3-Adamantan-1-yl-4-(3-ethoxymethoxypropyl)-phenyl]-propen-(Z)-yl}-benzoesäure;
- Ethyl-4-{2-[3-adamantan-1-yl-4-(3-benzyloxypropyl)-phenyl]-propen-(E/Z)-yl}-benzoat;
- 4-{2-[3-Adamantan-1-yl-4-(3-benzyloxypropyl)-phenyl]-propen-(E)-yl}-benzoesäure;
- 4-{2-[3-Adamantan-1-yl-4-(3-benzyloxypropyl)-phenyl]-propen-(Z)-yl}-benzoesäure;
- Ethyl-4-{2-[3-adamantan-1-yl-4-(3-diethylcarbamoylmethoxypropyl]-phenyl)-propenyl}-benzoat;
- 4-{2-[3-Adamantan-1-yl-4-(3-diethylcarbamoylmethoxypropyl)-phenyl]-propenyl}-benzoesäure;
- Ethyl-4-{2-[3-adamantan-1-yl-4-(3-carboxymethoxypropyl)-phenyl]-propenyl}-benzoat;
- 4-{2-[3-Adamantan-1-yl-4-(3-carboxymethoxypropyl)-phenyl]-propenyl}-benzoesäure;
- Ethyl-4-{2-[3-adamantan-1-yl-4-(3-carbamoylmethoxypropyl]-phenyl)-propenyl}-benzoat;
- 4-{2-[3-Adamantan-1-yl-4-(3-carbamoylmethoxypropyl)-phenyl]-propenyl}-benzoesäure;
- N-Ethyl-4-[(E)-2-(3-(1-adamantyl)-4-methoxyethoxymethoxyphenyl)-1-propenyl]-benzamid;
- 4-[(E)-2-(3-(1-Adamantyl)-4-methoxyethoxymethoxyphenyl)-1-propenyl]-benzamid;
- N-4-(Hydroxyphenyl)-4-[(E)-2-(3-(1-adamantyl)-4-methoxyethoxymethoxyphenyl)-1-propenyl]-benzamid;
- 4-[(E)-2-(3-(1-Adamantyl)-4-methoxyethoxymethoxyphenyl)-1-propenyl]-benzolmethanol;
- 4-[(E)-2-(3-(1-Adamantyl)-4-methoxyethoxymethoxyphenyl)-1-propenyl]-benzaldehyd;
- 4-[(E)-2-(3-(1-Adamantyl)-4-methoxyethoxymethoxyphenyl)-1-propenyl]-phenol;
- 4-[(E)-2-(3-(1-Adamantyl)-4-methoxyethoxymethoxyphenyl)-1-propenyl]-benzoesäure-Morpholid; und
- 4-[(E)-2-(3-(1-Adamantyl)-4-methoxyethoxymethylsulfanylphenyl)-1-propenyl]-benzoesäure,

8. Verbindungen nach Anspruch 1, dadurch gekennzeichnet, daß sie mindestens eine der folgenden Eigenschaften aufweisen:
- R₁ ist eine Gruppe -CO-R₇,
- Ar bedeutet eine Gruppe der Formel (a) oder (b).
- X und Y, die identisch oder voneinander verschieden sind, bedeuten Sauerstoff oder Schwefel, und
- R₃ ist eine Alkylgruppe, die unter Methyl, Ethyl, Propyl, Isopropyl, *tert.*-Butyl oder Hexyl ausgewählt ist.

9. Verbindungen nach einem der vorhergehenden Ansprüche zur Verwendung als Arzneimittel.

10. Verwendung einer Verbindung nach einem der Ansprüche 1 bis 8 zur Herstellung eines Arzneimittels, das zur Behandlung der folgenden Erkrankungen vorgesehen ist:
- Acne vulgaris, Acne comedonica, polymorpher Acne, Acne rosacea, nodulocystischer Acne, Acne conglobata, Acne senilis, sekundären Acneformen, Acne solaris, Acne medikamentosa oder Acne professionalis;
- Ichtyosis, ichtyosisartigen Zuständen, der Darier Kankheit, Palmoplantarkeratosen, Leukoplakien und leucoplakieformen Zuständen und Lichen der Haut oder der Schleimhäute (buccalis);
- Psoriasis der Haut, der Schleimhäute oder der Nägel, Psoriasis anthropathica, Atopie der Haut, Ekzemen, Atopie der Atemwege oder auch Hypertrophie des Zahnfleisches;
- Proliferationen der Dermis oder Epidermis, die gutartig oder bösartig und die gegebenenfalls viralen Ursprungs sein können, einschließlich Verrucae vulgares, Verrucae planae und Epidermodysplasia verruciformis, Papillomatosis oralis oder florida, und Proliferationen, die durch UV-Strahlung hervorgerufen werden können, einschließlich Epithelioma basocellulare und spinocellulare;
- bullösen Dermatosen und Erkrankungen des Kollagens;
- Corneopathien;
- Hautalterung, die lichtinduziert oder altersbedingt sein kann;
- Pigmentierungen und aktinischen Keratosen, oder beliebiger Erkrankungen, die mit der altersbedingten oder aktinischen Hautalterung verbunden sind;
- Atrophie der Haut;
- Wundmalen der epidermalen und/oder dermalen Atrophie, die durch lokal oder systemisch verabreichte Corticosteroide hervorgerufen wird;
- Störungen der Wundheilung oder Streifen;
- Funktionsstörungen der Talgdrüsen, einschließlich Hyperseborrhoe bei Acne oder Seborrhoe simplex;
- krebsartigen oder präcancerösen Zuständen;
- promyelocytärer Leukämie;
- Arthritis;
- Kaposi-Syndrom;
- Alopezie;
- Arteriosklerose oder Bluthochdruck sowie nicht insulinpfichtiger Diabetis; und
- Hautstörungen, die von einer Exposition gegenüber UV-Strahlung herrühren.

11. Verwendung einer Verbindung nach einem der Ansprüche 1 bis 8 zur Herstellung eines Arzneimittels, das zur Vorbeugung einer der folgenden Erkrankungen vorgesehen ist:
- Atopie der Haut, Ekzemen, Atopie der Atemwege oder Hypertrophie des Zahnfleisches;
- Hautalterung, die lichtinduziert oder altersbedingt sein kann;
- Atrophie der Haut;
- Wundmalen der epidermalen und/oder dermalen Atrophie, die durch lokal oder systemisch verabreichte Corticosteroide hervorgerufen wird;
- Störungen der Wundheilung;
- Streifen;
- krebsartigen oder präcancerösen Zuständen;
- promyelocytärer Leukämie;
- Alopezie;
- Arteriosklerose oder Bluthochdruck sowie nicht insulinpfichtiger Diabetis; und
- Hautstörungen, die von einer Exposition gegenüber UV-Strahlung herrühren.

12. Pharmazeutische Zusammensetzung, dadurch gekennzeichnet, daß sie in einem pharmazeutisch akzeptablen Träger mindestens eine Verbindung nach einem der Ansprüche 1 bis 8 enthält.

13. Zusammensetzung nach Anspruch 12, dadurch gekennzeichnet, daß sie die Konzentration der Verbindung(en) nach einem der Ansprüche 1 bis 8 im Bereich von 0,001 bis 5 Gew.-%, bezogen auf die gesamte Zusammensetzung, liegt.

14. Kosmetische Zusammensetzung, dadurch gekennzeichnet, daß sie in einem kosmetisch akzeptablen Träger mindestens eine Verbindung nach einem der Ansprüche 1 bis 8 enthält.

15. Zusammensetzung nach Anspruch 14, dadurch gekennzeichnet, daß sie die Konzentration der Verbindung(en) nach einem der Ansprüche 1 bis 8 im Bereich von 0,001 bis 3 Gew.-%, bezogen auf die gesamte Zusammensetzung, liegt.

16. Verwendung einer kosmetischen Zusammensetzung nach einem der Ansprüche 14 oder 15 zur Körper- oder Haarhygiene.
